# EUROPEAN PATENT APPLICATION

(11) **EP 2 617 809 A2**
(43) Date of publication of application: **24.07.2013**
(21) Application number: 13156244.9
(22) Date of filing: 27.06.2008
(51) Int. Cl.: C12N 5/07

(54) **Neural stem cells**

(30) Priority: 27.06.2007 US 937571 P
(62) Divisional of application: 08768833.9
(71) Applicant: President and Fellows of Harvard College, Cambridge, MA 02138 (US)
(72) Inventor: Cardozo, David L, Newton, MA 02461-1014 (US); Jha, Ruchira, Boston, MA 02114 (US)
(74) Representative: Wainwright, Jane Helen

(57) **Abstract**

The invention provides compositions and methods for obtaining neural stem cells from post-natal subjects and their use in treating neurological disorders.

## Description

### RELATED APPLICATIONS

This application is related to provisional application USSN 60/937,571, filed June 27, 2007, the contents of which are herein incorporated by reference in their entirety.

### FIELD OF THE INVENTION

The present invention relates to the field of cell therapy.

### BACKGROUND OF THE INVENTION

Neural stem cells (NSC) and progenitor cells are developmentally primitive cells that reside in the central nervous system (CNS) and are capable of generating all of the major cell types therein: neurons, astrocytes, and oligodendrocytes. NSCs are useful for the study of human nervous development and neurological diseases as well as for treating such diseases.

NSCs have been harvested from areas deep within the brain, e.g., from the subventricular zone of the lateral ventricles and the granule cell layer of the hippocampus. The potential for damage to overlying brain areas during the harvest of NSCs from these regions makes this strategy for the harvest of autologous NSCs dangerous and impractical.

### SUMMARY OF THE INVENTION

The present invention addresses these difficulties by identifying a population of NSCs that are readily accessible and describing a method for isolating them that poses far less risk of injury compared to existing methods. The cells are allogeneic or autologous. To avoid complications due to transplantation of heterologous tissue, NSCs are preferably autologous. For example, a pharmaceutical composition for cell replacement or tissue regeneration contains a population of filum terminale (FT) neural cells enriched for NSCs. The population contains a neurosphere or a neurosphere initiating cell (NS-IC). Neurospheres are aggregates or clusters of cells that contain neural stem cells. They are typically spherical in nature and free-floating in culture. Cells in the neurospheres proliferate in culture while retaining the potency to differentiate into neurons and glia. A NS-IC is a cell that can initiate long-term neurosphere culture. A NS-IC is nestin-positive and has the capability to differentiate, under appropriate differentiating conditions, to neurons, astrocytes, and oligodendrocytes. Preferably, the composition comprises a population of isolated FT cells at least 10% of which are NSCs or NS-ICs. For example, at least 30%, 50%, 85%, 90%, 99% or 100% of the cell population are NSCs.

FT, a dispensable neural tissue, is harvested from a subject, e.g., a human patient suffering from or at risk of developing a neurological injury or other disorder. Isolation of NSCs from a post-natal animal is carried out by providing a FT tissue from the subject, dissociating the FT tissue to obtain neurospheres, and recovering nestin-positive NSCs. A composition containing an isolated NSC obtained in this manner, i.e., a population of autologous NSC, is used to treat the subject from which the tissue was obtained. A neural stem cell obtained from FT tissue is referred to as FT-NSC. Also within the invention is a cell line containing multipotent descendant cells of an FT-NSC.

Accordingly, a method of augmenting or restoring neurological function in a subject is carried out by administering to the subject a population of isolated FT cells. The cells are administered to a subject, e.g., the cells are implanted locally directly into the site of injury or damage or administered to a site that is remote from the affected site. For example, cells are introduced intraventricularly to a damaged portion of the brain or infused into spinal fluid. A method of treating a neurological disorder includes the steps of harvesting FT tissue from a subject, culturing FT cells *ex vivo* to produce an enriched population of isolated FT-NSCs, and administering to the subject the enriched population of isolated FT-NSCs. Neurological disorders to be treated include an injury (acute or chronic) or a degenerative condition. A neurological disorder includes an injury or diminuition of function of the brain or spinal cord regardless of origin of the defect. For example, the subject is diagnosed as having suffered a stroke or suspected of having suffered a stroke.

The cells are used to reconstitute neural tissue that has been lost through disease or injury. Genetic diseases associated with neural cells may be treated by genetic modification of autologous or allogeneic stem cells to correct a genetic defect or treat to protect against disease. CNS disorders to be treated include neurodegenerative diseases (e.g. Alzheimer's Disease, Multiple Sclerosis (MS), Huntington's Disease, Amyotrophic Lateral Sclerosis, and Parkinson's Disease), acute brain injury (e.g. stroke, head injury, cerebral palsy) as well as other CNS dysfunctions (e.g. depression, epilepsy, and schizophrenia).

Also within the invention is a method of expanding FT-NSCs from a post-natal animal by providing isolated FT-NSC from the animal and culturing the FT-NSC under conditions that allow for proliferation or differentiation of the FT-NSC.

The compositions described herein are purified or isolated. By "substantially pure" is meant a nucleic acid, polypeptide, or other molecule that has been separated from the components that naturally accompany it. Typically, the polypeptide is substantially pure when it is at least 60%, 70%, 80%, 90%, 95%, or even 99%, by weight, free from the proteins and naturally-occurring organic molecules with which it is naturally associated. For example, a substantially pure polypeptide may be obtained by extraction from a natural source, by expression of a recombinant nucleic acid in a cell that does not normally express that protein, or by chemical synthesis. The term "isolated nucleic acid" is meant DNA that is free of the genes which, in the naturally occurring genome of the organism from which the given nucleic acid is derived, flank the DNA. Thus, the term "isolated nucleic acid" encompasses cloned nucleic acids or synthetic nucleic acids (RNA, RNAi, DNA).

An effective amount is an amount of a composition, e.g., a cell sample, required to confer clinical benefit. The effective amount varies depending upon the route of administration, age, body weight, and general health of the subject. A pharmaceutical composition is a composition, which contains at least one therapeutically or biologically active agent and is suitable for administration to the patient. Such compositions are prepared by well-known and accepted methods of the art. See, for example, Remington: The Science and Practice of Pharmacy, 20th edition, (ed. A. R. Gennaro), Mack Publishing Co., Easton, Pa., 2000. Parenteral administration, such as intravenous, subcutaneous, intramuscular, and intraperitoneal delivery routes, may be used to deliver the pharmaceutical compositions. Alternatively, the compounds are administered locally, e.g., directly to a CNS site. For treatment of neurological disorders, direct infusion into cerebrospinal fluid or direct injection into brain tissue is used. Dosages for any one patient depends upon many factors, including the patient's size, body surface area, age, the particular nucleic acid to be administered, sex, time and route of administration, general health, and other drugs being administered concurrently. For example, a concentrated cell suspension containing approximately 5 x 10⁵ - 1x 10⁶ cells are injected into a site. For treatment of Parkinson's Disease, the cells are injected into the wall of a ventricle.

Other features and advantages of the invention will be apparent from the following description of the preferred embodiments thereof, and from the claims. References cited are hereby incorporated by reference.

### BRIEF DESCRIPTION OF THE DRAWINGS

Figure 1A is a diagram of a sagittal cross-section showing the FT relative to lumbar and sacral vertebrae (left panel) and a diagram showing the de-differentiation of the caudal spinal cord into the FT during embryonic development (middle and right panels).

Figure 1B is a dissected 17.5 week human fetal spinal cord. Arrow indicates FT. Scale Bar=2.5 mm.

Figure 1C is a merged image from a portion of a transverse section of 8 month old *FT,* stained for NSC marker Nestin (red) and DAPI (blue). Scale Bar= 100 µm.

Figure 2A is a fluorescence micrograph showing the presence of nestin-expressing neural stem cells (green) in a neurosphere derived from an embryonic human (week 17) FT after 7 DIV.

Figure 2B is a fluorescence micrograph showing the continued presence of nestin-expressing neural stem cells (red) in a neurosphere derived from a postnatal day (P) 5 rat *filum terminale* after 35 DIV.

Figure 3A is a phase-contrast micrograph showing cells taken from a neurosphere derived from 6-month old human. *filum terminale.*

Figure 3B is a fluorescence micrograph showing neurons expressing beta-III tubulin in adhesive culture after 18 DIV. The cells were taken from a neurosphere derived from 12-year-old human *filum terminale.*

Figure 3C is a fluorescence micrograph showing oligodendrocytes expressing O1 (green) in culture after 18 DIV. The cells came from neurospheres derived from a P6 rat *filum terminale.* Cell nuclei are counterstained with DAPI (blue).

Figure 3D is a fluorescence micrograph showing the coexistence of vimentin expressing neural precursor cells (red) and GFAP expressing astroglia (green) in adherent cultures of neurosphere derived cells. The neurospheres themselves were derived from the *filum terminale* of a P4 rat. Nuclei are counterstained with DAPI (blue).

Figure 4A is a fluorescence micrograph showing beta-III tubulin (green) expressing motomeurons following exposure of neurosphere derived cells to retinoic acid and sonic hedgehog alongside GDNF, BDNF and CNTF. The cells were derived from neurospheres taken from P7 rat *filum terminale*.

Figure 4B is a fluorescence micrograph of the same field of cells in 4(A) showing motomeuron expression of Choline Acetyltransferase (red).

Figure 4C is a fluorescence micrograph of a different neurosphere showing beta-III tubulin expressing motomeurons (red).

Figure 4D is a fluorescence micrograph of the same field of cells showing simultaneous expression of MCN-2, a marker uniquely expressed by motomeurons. All cells were derived from the *filum terminale* of P7 rats. These figures depict directed differentiation of cells from *filum terminale* neurospheres into motor neurons

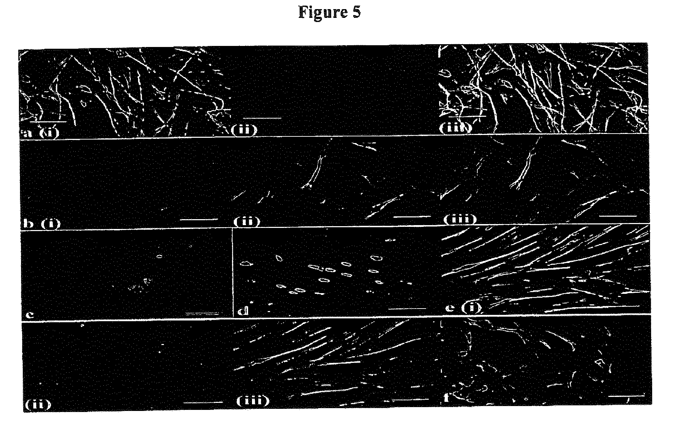

Figures 5A-F are fluorescence micrographs showing neurospheres differentiated into neural progenitor cells (NPC), neurons and/or glia. a) Differentiated cells derived from a single neurosphere stained for Vimentin (i, green), Tuj-1 (ii, red), and merged image (iii). Plated on poly-L-lysine and laminin in 5% serum for 7 days. Donor: 12 years old. 18 days in *vitro.* b) Differentiated cells from a single neurosphere expressing GFAP (i, red) and Tuj-1 (ii, green). The merged image is shown in (iii). Same donor and conditions as (a). 17 days *in vitro.* c) Phase microscopy showing silver grains for the ³[H]-thymidine labeled nuclei of neurons differentiated from FT neurospheres in 5% serum over 7 days. Neurospheres were exposed to ³[H]-thymidine for 8 hours prior to culture in differentiating conditions. Cells are counterstained for Tuj-1 (green). Donor: 6 month FT, 107 *days in vitro*. d, e) Differentiated cells from FT neurospheres stained for MN markers after treatment with RA and Shh-N. d) Differentiated cells stained for Tuj-1 (red) and MNR-2 (green). Donor: 14 week fetus, 81 *days in vitro.* e) Differentiated cells stained for Tuj-1 (i, green), choline-acetyltransferase (ii, red). Merged image is shown in (iii). Donor: 18 year old, 25 days *in vitro.* f) Differentiated cells stained for O1 (green) and counterstained for DAPI. Donor: 6 month old, 111 days *in vitro.* Scale bars: a-f = 50 um.

Figure 6A is a photomicrograph of the caudal aspect of the rat spinal cord at P7 showing the FT. The arrow indicates the portion of the structure used to generate neurospheres.

Figure 6B is a fluorescence micrograph of a P7 FT tissue section showing (i) expression of the NSC-marker Nestin (red); (ii) DAPI counterstaining (blue); and (iii)the merged image. Scale bar is 100 um.

Figure 6C is a photomicrograph showing a neurosphere derived from the P10 rat FT after 10 days *in vitro.* Scale bar is 100 um.

Figure 6D is a fluorescence micrograph of a neurosphere derived from a P5 FT after 5 days *in vitro* showing (i) Nestin expression; (ii) DAPI counterstaining, and (iii) the merged image. Scale bar is 100 um.

Figure 7A is a fluorescence micrograph of a neurosphere derived from P7 FT after 34 days *in vitro* showing (i) expression of the NPC marker Olig2 (red); (ii) DAPI counterstain (blue); and (iii) the merged image.

Figure 7B is a fluorescence micrograph of a neurosphere derived from P7 FT after 60 DIV showing (i) expression of Vimentin (green); (ii) DAPI counterstain (blue); and (iii) the merged image.

Figure 7C is a fluorescence micrograph of a neurosphere isolated from P6 rFT after 30 DIV showing (i) expression of Sox2 (red) in some cells; (ii) DAPI counterstain (blue); and (iii) the merged image.

Figure 7D is a fluorescence micrograph of a neurosphere derived from P6 FT after 30 DIV showing (i) Weak staining of Musashi (COLOR); (ii) DAPI counterstain (blue); and (iii) the merged image.

Figure 7E is a fluorescence micrograph of a neurosphere derived from P7 FT after 34 DIV showing (i) expression of beta-III-tubulin (COLOR); (ii) expression of GFAP (COLOR); (iii) DAPI counterstain (blue); and (iv) the merged image

Figure 8A is a fluorescence micrograph showing (i) Pax6 expression (green); (ii) Olig2 expression (red); (iii) DAPI counterstain (blue); and (iv) the merged image. Scale bar is 100 um. Cells derived from P7 rat FT at 30 DIV The data indicate that motor neurons are generated from FT-derived neurospheres.

Figure 8B is a similar fluorescence micrograph showing (i) Pax6 expression (green); (ii) Olig2 expression (red); (iii) DAPI counterstain (blue); and (iv) the merged image. Scale bar is 50 um. Cells derived from P7 rat FT at 30 DIV.

Figure 8C is a fluorescence micrograph showing (i) expression of Beta-III tubulin (green), an early neuronal marker; (ii) ChAT (red), a marker of cholinergic neurotransmission; (iii) DAPI counterstain (blue); and (iv) the merged image. Scale bar is 100 um. Donor: P6 rat FT 36 DIV.

Figure 8D is a fluorescence micrograph showing expression of (i) MNR2 (green), a motor neuron specific marker; (ii) beta-III-tubulin (red), an early neuronal marker; (iii) DAPI counterstain; and (iv) the merged image. Scale bar is 50 um. Donor: P7 FT, 30 DIV.

Figure 8E is a fluorescence micrograph of an undissociated neurosphere culture showing non-overlapping expression of (i) MRN2 (green); (ii) GFAP (red); (iii) DAPI counterstain; and (iv) the merged image. These cultures were not treated with Sonic Hedgehog and Retinoic Acid; rather, they were maintained solely in differentiating medium containing BDNF, CNTF & GDNF. Scale bar is 50 um. Donor: P7 FT, 30 DIV.

Figure 8F is a fluorescence micrograph of another undissociated neurosphere cultured under the same conditions as 7 E showing an island of motorneurons among GFAP-expressing cells. The panel show expression of (i) MRN2 (green); (ii) GFAP (red); (iii) DAPI counterstain; and (iv) the merged image. Scale bar is 50 um. Donor: P7 FT, 30 DIV.

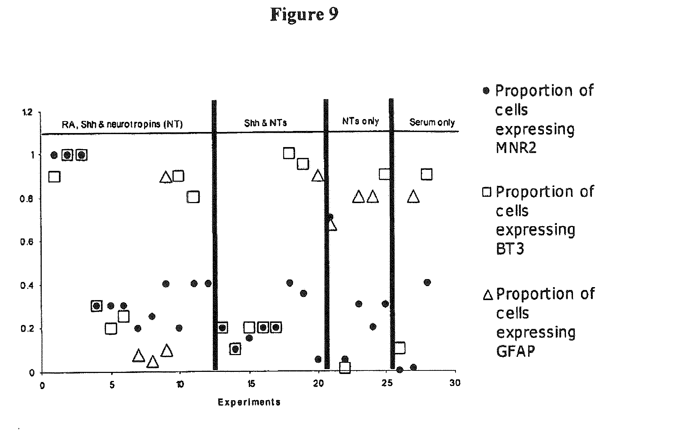

Figure 9 is a scatter-plot summarizing the results of 28 directed differentiation experiments using rat FT NSCs, showing increased rates of MRN2 expression in cultures treated with retinoic acid (RA), Sonic Hedgehog (SHH), BDNF, CNTF and GDNF. Note that MNR2 was also expressed in differentiated cells from neurospheres treated with Shh alone, and in some cases from untreated neurospheres grown in the presence of BDNF, CNTF, and GDNF alone. In 1 of 3 cases, immunostaining for MNR2 was observed in 40% of cells from untreated neurospheres differentiated in serum without any specific neurotrophic factors.

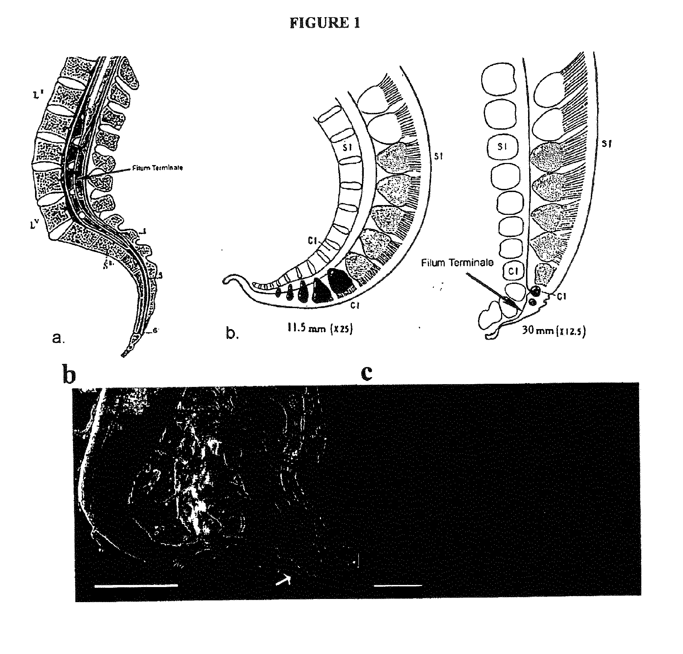

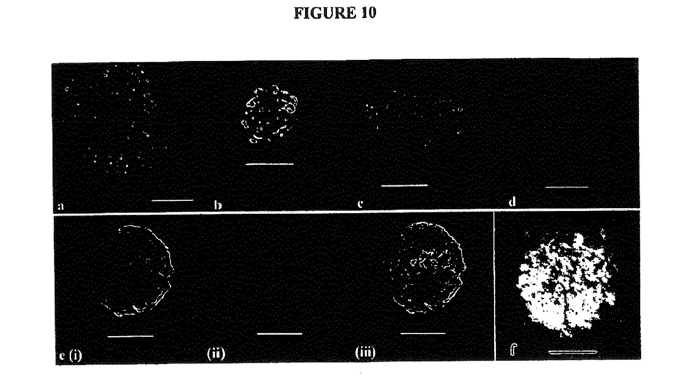

Figures10A-F are fluorescent micrographs of neurospheres stained for cell markers to characterize cell-type expression. a) Neurosphere derived from the same 8 month FT as Figure 1(c) stained for Nestin (red), 11 days *in vitro.* b) Neurosphere stained for Vimentin (green). Donor: 6 month FT, 123 days *in vitro.* c) Olig-2 staining (red) in two neurospheres from same donor as (b). d). Sox-2 expression (red) in a neurosphere derived from a 10 year old. 51 days *in vitro.* e) Tuj-1 (i, green), GFAP (ii, red), merged image (iii) of a neurosphere from the same donor as (a). f) Phase contrast micrograph of neurosphere from 18 year old *FT.* 6 *days in vitro.* Scale bars: a-f = 100 µm. Images a-d & e (iii) are counterstained with DAPI (blue).

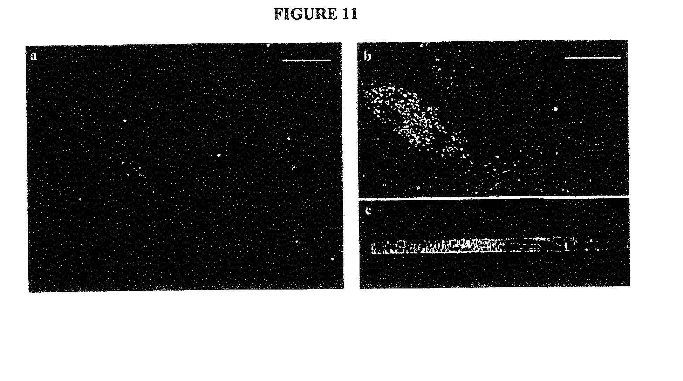

Figures 11A-C are fluorescent micrographs of neuromuscular junctions showing derived human MN and rat muscle co-culture stained for α-Bungarotoxin (green) and Tuj-1 (red). A single neurosphere differentiated with RA and Shh-N was co-cultured with rat muscle fibers for 6 days. a) Fluorescent micrograph of MN (red) and α-Bungarotoxin labeled acetylcholine receptors (green). b) Confocal microscopy image of the neuromuscular junction shown in (a). c) Side view of (b) processed into a maximum intensity projection (MIP) rendering in Imaris Surpass. The nerve terminal (red) lies above, the labeled receptors (green) below and yellow indicates the area of overlap. Donor: 6 month old FT. Scale bars 50 µm.

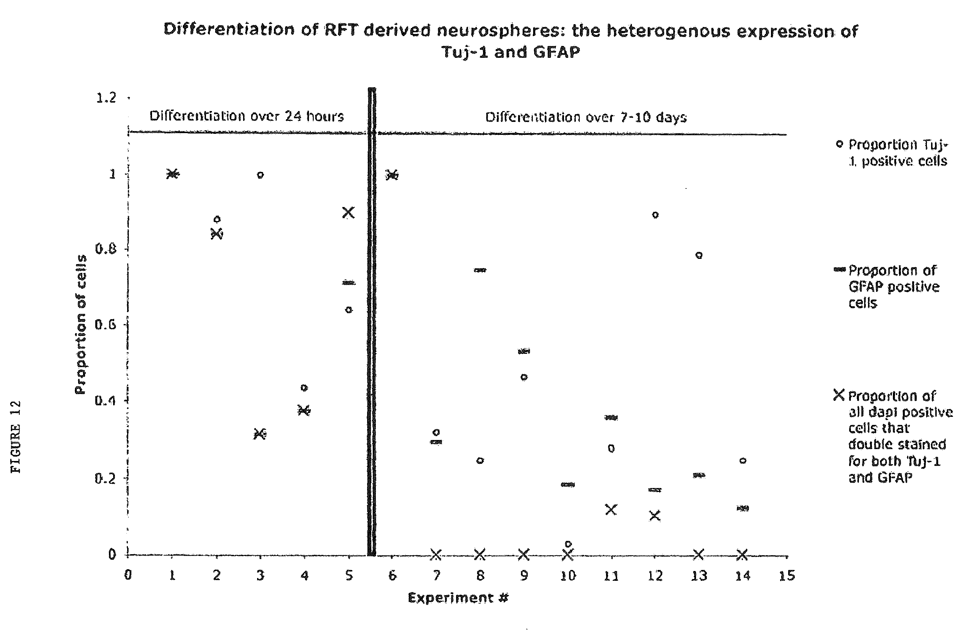

Fig. 12 is a scatter graph showing heterogeneous differentiation potential of rFT derived neurospheres. The scatter graph illustrates the variability in expression of the Tuj-1 (neuronal marker) and GFAP (astrocytic marker) in 14 experiments of neurosphere differentiation. Individual neurospheres were differentiated by plating them on poly-L-lysine and laminin coated coverslips and culturing them in 5% serum. Differentiated cells were evaluated by immunocytochemistry after either 24 hours, or after 7-10 days. In both cases, there was a variable generation of neurons and astrocytes. The approximate proportion of differentiated cells from a neurosphere that double stained for both markers decreased after 7.-10 days of exposure to the differentiating conditions relative to the proportion of overlap noted after 24 hours.

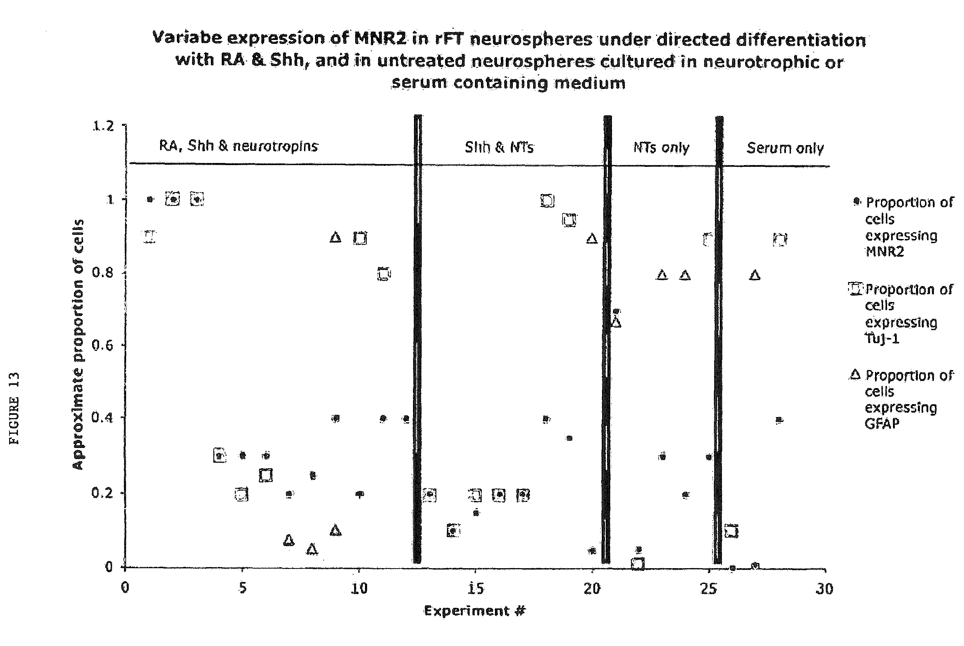

Fig. 13 is a scatter graph showing variable expression of MNR2 in rFT derived neurospheres undergoing MN differentiation. The scatter graph illustrates the variability in MNR2 expression in differentiated neurospheres. MNR2 was one of the multiple markers used to identify MN generation. As evident in the chart, MNR2 was also expressed in differentiated cells from neurospheres treated with Shh alone, and in those from untreated neurospheres grown in the presence of BDNF, CNTF, and GDNF. In 1 of 3 cases, immunostaining for MNR2 was observed in '40% of cells from untreated neurospheres differentiated in serum without any specific neurotrophic factors.

### DETAILED DESCRIPTION

The invention represents a major advance in stem cell-based therapy. The field has been stymied by a shortage of sources and reliable methods of procurement of stem cells for therapeutic purposes. Particularly difficult has been the procurement of neural stem cells for use in the replacement of brain cells (neurons and glia) that are lost due to brain or spinal cord injury or degenerative disease.

A completely novel source for autologous human neural stem cells has been identified. The FT sits at the caudal end of the spinal cord attaching the cord to the coccygeal bone. It is a structurally distinct anatomical tissue that is separate from the conus medullaris, e.g., in an adult human, the tissues are separated by 2-3 cm. It is a vestigial tissue expendable in the nervous system. In contrast to other sources of neural stem cells, the FT is surgically accessible and tissue is routinely obtained from it during operations for "tethered cord syndrome". It is a reliable source for neural stem cells. Stem cells obtained from this source can be grown and maintained over long periods of time in tissue culture and have been demonstrated to differentiated into neurons and glia. FT NSC are easily harvested from patients suffering from neurological injury or degeneration, expanded and differentiated in tissue culture and subsequently transplanted back into the patient. This strategy overcomes the problems of tissue rejection that accompany transplantation of non-autologous cells. The FT represents the first easily-accessible, expendable nervous tissue that serves as a source of cells suitable for nerve cell replacement strategies.

### Isolation of FT

The invention provides compositions and methods that increase the feasibility of NSC therapy through isolation of neural stem cells from FT, an area of the central nervous system that has never been investigated for the presence of stem cells. Previous work has concentrated on the isolation and proliferation of NSCs from known regions within the CNS that contain stem cells, which are essential to normal brain function and difficult to access surgically. The FT is a novel and attractive alternative to previously used sources of NSCs because the FT is a histologically primitive structure that represents a non-function remnant of the developing spinal cord in post-natal mammals. These properties make the FT an attractive source for autologous NSCs for therapeutic use. FT are obtained from postnatal mammals (for instance rats or humans), discarded human fetuses, human from children and adolescents (especially those who have undergone surgical resection for tethered spinal cords), and any post-natal subject in need of neural stem cells for therapeutic use. The tissue is dissociated and neurospheres are isolated and grown under conditions that promote stem cell survival and/or proliferation. FT-derived cells are administered to patients in need of restoration of neurological function due to a developmental or degenerative condition or disorder, disease, injury or trauma, infection, complication from medication or a medical procedure, or any other natural (e.g. aging) or induced (e.g. stroke) cause. Transplanted cells integrate into the host central or peripheral nervous system and to promote functional recovery.

### Stem Cells

Stem cells are cells found in most, if not all, multi-cellular organisms. They are characterized by the ability to renew themselves through mitotic cell division and differentiating into a diverse range of specialized cell types. The two broad types of mammalian stem cells are: embryonic stem cells that are found in blastocysts, and adult stem cells that are found in adult tissues. In a developing embryo, stem cells can differentiate into all of the specialized embryonic tissues. In adult organisms, stem cells and progenitor cells act as a repair system for the body, replenishing specialized cells, but also maintain the normal turnover of regenerative organs, such as blood, skin or intestinal tissues.

As used herein, the term "stem cell" is meant to describe a cell which is capable of self-renewal and is capable of differentiating into more than one type of cell. Self-renewal is defined herein as the ability to go through numerous cycles of cell division while maintaining an undifferentiated state.

Potency is the capacity to differentiate into specialized cell types. In the strictest sense, this requires stem cells to be either totipotent or pluripotent - to be able to give rise to any mature cell type, although multipotent or unipotent progenitor cells are sometimes referred to as stem cells. In other terms, potency specifies the differentiation potential (the potential to differentiate into different cell types) of the stem cell. Totipotent stem cells are produced from the fusion of an egg and sperm cell. Cells produced by the first few divisions of the fertilized egg are also totipotent. These cells can differentiate into embryonic and extraembryonic cell types. Pluripotent stem cells are the descendants of totipotent cells and can differentiate into cells derived from any of the three germ layers. Multipotent stem cells can produce only cells of a closely related family of cells (e.g. hematopoietic stem cells differentiate into red blood cells, white blood cells, platelets, etc.). Unipotent cells can produce only one cell type, but have the property of self-renewal which distinguishes them from non-stem cells (e.g. muscle stem cells).

Stem cells of the invention are identified by molecular and functional methods. The practical definition of a stem cell is the functional definition - the ability to regenerate tissue over a lifetime. Properties of stem cells can be illustrated *in vitro,* using methods such as clonogenic assays, in which single cells are characterized by their ability to differentiate and self-renew. Moreover, stem cells and stem cell populations are identified and isolated based on a distinctive set of cell surface and intracellular markers.

Embryonic stem cell lines (ES cell lines) are cultures of cells derived from the epiblast tissue of the inner cell mass (ICM) of a blastocyst or earlier morula stage embryos. A blastocyst is an early stage embryo-approximately four to five days old in humans and consisting of 50-150 cells. ES cells are pluripotent and give rise during development to all derivatives of the three primary germ layers: ectoderm, endoderm and mesoderm. In other words, they can develop into each of the more than 200 cell types of the adult body when given sufficient and necessary stimulation for a specific cell type.

After nearly ten years of research, there are no approved treatments or human trials using embryonic stem cells. ES cells, being totipotent cells, require specific signals for correct differentiation - if injected directly into another body, ES cells will differentiate into many different types of cells, causing a teratoma (i.e. a type of neoplasm). Differentiating ES cells into usable cells while avoiding transplant rejection are just a few of the hurdles that embryonic stem cell researchers still face. Many nations currently have moratoria on either ES cell research or the production of new ES cell lines.

As used herein, the term "adult stem cell" refers to any cell which is found in a developed organism that has two properties: the ability to divide and create another cell like itself (i.e. self-renew) and also divide and create a cell more differentiated than itself (i.e. the cell is at least unipotent, but preferentially, at least multipotent). Adult stem cells are also commonly known as somatic stem cells and germline stem cells. Adult stem cells can be identified in all postnatal mammals.

Pluripotent adult stem cells can be found in a number of tissues including umbilical cord blood. Most adult stem cells are lineage-restricted (multipotent) and are generally referred to by their tissue origin (mesenchymal stem cell, adipose-derived stem cell, endothelial stem cell, etc.).

In one aspect, to ensure self-renewal, stem cells undergo two types of cell division that differ by plane of division and/or division of intracellular elements. Symmetric division gives rise to two identical daughter cells both endowed with stem cell properties. Symmetric division is defined as cell division that produces two identical cells, cell division that occurs with a plane of division parallel to an epithelial barrier (the lateral ventricle of the cerebral hemispheres for instance), cell division that produces two evenly sized cells or occurs at a center point (equator) of a cell, or cell division that produces two cells with the same or equivalent intracellular components/elements following separation. Asymmetric division, on the other hand, produces only one stem cell and a progenitor cell with limited self-renewal potential. Asymmetric division is defined as cell division that is asymmetric for all of the above-listed scenarios. Exemplary intracellular components or elements that may be symmetrically or asymmetrically divided between daughter cells include, but are not limited to, intracellular or cell-surface proteins; cytoskeletal elements; adherins junctions, cell-contact, or cell-adhesion elements; intracellular organelles; and signaling molecules. Progenitors can go through several rounds of cell division before terminally differentiating into a mature cell.

An alternative theory is that stem cells remain undifferentiated due to environmental cues in their particular niche. Stem cells differentiate when they leave that niche or no longer receive those signals. *In vitro*, a stem cell niche can be recapitulated to induce directed *ex vivo* stem cell differentiation. In certain aspects, particular components of stem cell niches can be overexpressed, removed, recombined, and/or synthesized to induce desired stem cell responses.

### Neural Stem Cells

As used herein, the term "neural stem cell" is meant to describe a stem cell found in adult neural tissue that can give rise to neurons and glial cells. Examplary glial cells include, but are not limited to, astrocytes and oligodendrocytes. Neurons (also known as neurones and nerve cells) are electrically excitable cells in the nervous system that process and transmit information. Neurons are the core components of the brain, spinal cord, and peripheral nerves in vertebrates. Fully developed neurons are permanently amitotic (they do not divide); however, additional neurons throughout the brain can originate from neural stem cells found in the subventricular zone and subgranular zone through the process of neurogenesis. The instant invention also provides a source of neural stem cells in the spinal cord, with the FT.

### Neurons

Neurons are typically composed of a soma, or cell body, a dendritic tree and an axon. The majority of vertebrate neurons receive input on the cell body and dendritic tree, and transmit output via the axon. However, there is great heterogeneity throughout the nervous system and the animal kingdom, in the size, shape and function of neurons. Some specialized neuronal subtypes are known including, but not limited to, Basket cells (neurons with dilated and knotty dendrites in the cerebellum); Betz cells (large motor neurons); Medium spiny neurons (most neurons in the corpus striatum); Purkinje cells (huge neurons in the cerebellum, a type of Golgi I multipolar neuron); Pyramidal cells (neurons with triangular soma, a type of Golgi I); Renshaw cells (neurons with both ends linked to alpha motor neurons); Granule cells (a type of as Golgi II neuron); and anterior hom cells (motoneurons located in the spinal cord). Due to the wide variation of neuronal subtypes, mature neurons of the invention are identified using one or more methods that exploit morphological, molecular, and/or functional differences between cell types.

Differentiated neurons are characterized by their actions on other neurons or cells. Excitatory neurons excite their target neurons. Excitatory neurons in the central nervous system, including the brain, are often glutamatergic. Neurons of the peripheral nervous system, such as spinal motoneurons that synapse onto muscle cells, often use acetylcholine as their excitatory neurotransmitter. Inhibitory neurons inhibit their target neurons. Inhibitory neurons are often interneurons. The output of some brain structures (neostriatum, globus pallidus, cerebellum) are inhibitory. The primary inhibitory neurotransmitters are GABA and glycine. Modulatory neurons evoke more complex effects termed neuromodulation. These neurons use such neurotransmitters as dopamine, acetylcholine, serotonin and others.

Differentiated neurons are characterized by their discharge patterns. Neurons are classified according to their electrophysiological characteristics. Neurons display tonic or regular "spiking," e.g., a spike refers to the detection of an action potential. Some neurons are typically constantly (or tonically) active, e.g. intemeurons in neurostriatum. Other neurons display regular spiking which refers to action potentials that are evoked by at least one stimulus. Alternatively, or in addition, neurons display phasic or bursting behavior. Neurons that fire in bursts are called phasic. Some neurons are notable for their fast firing rates, for example some types of cortical inhibitory interneurons, cells in globus pallidus. Alternatively, or further in addition, action potentials of some neurons are more narrow compared to the others (thin-spikes when detected). For example, interneurons in prefrontal cortex are thin-spike neurons.

Differentiated neurons are characterized by the neurotransmitter they release. Non-limiting exemplary neuronal types are cholinergic neurons, GABAergic neurons, glutamatergic neurons, dopaminergic neurons, and 5-hydroxytryptamine neurons (5-HT; serotonin).

### FT-NSC Characterization

To further characterize the cells, FT-NSC lines are established and maintained. Neurospheres derived from FT are examined for the expression of the NSC markers Vimentin, CD 133, Olig2, and Sox 2. FT-NSCs are cultured *in vitro* under conditions that promote differentiation. Differentiated cell types are identified using both immunocytochemical techniques and electrophysiological methods. Rat and human FT-NSCs are transplanted into the CNS of either normal rodents or rodents that model spinal cord trauma or neurological disease.

FT-NSCs have been isolated from rats and humans (approximately 24 human donors), and maintained as neurospheres over many passages in tissue culture. For example, cells have been passaged 15-20 times and some cell lines have been maintained for over 12 months. FT-NSCs obtained in this manner were induced to differentiate into CNS neurons and glia as determined by immunocytochemistry. The cells give rise to both neurons and glia (both astrocytes and oligodendrocytes). The cells have been induced to differentiate to yield a cell population that is 80-100% motor neurons.

### Identification of a source of autologous stem cells

A source of human NSCs was identified and their therapeutic use in cases of nervous system trauma and degeneration studied. Autologous NSCs are isolated from the FT of the spinal cord, expanded and subsequently transplanted to a site of nerve tissue damage.

A reliable source of autologous of NSCs is the FT of the spinal cord, a slender prolongation of the caudal end of the spinal cord that anchors the cord to the coccyx at the base of the spine (Fig. 1A). Histological studies show that FT encloses a ventricular canal surrounded by peri-ventricular ependymal cells as well as various types of neurons and glia. This local environment is similar to other CNS regions that produce NSCs (Alvarez-Buylla, A. and D.A. Lim. Neuron, 2004. 41(5): p. 683-6; Doetsch, F. Curr Opin Genet Dev, 2003. 13(5): p. 543-50; Riquelme, P.A., E. Drapeau, and F. Doetsch. Philos Trans R Soc Lond B Biol Sci, 2007). FT is not interconnected with the rest of the nervous system nor does it innervate the body. In essence, it is an expendable remnant of the nervous system.

The FT has a unique developmental history (Streeter, G.L. Am J Anat, 1919. 22: p. 1-12; Nievelstein, R.A., et al. Teratology, 1993. 48(1): p. 21-31; Kemohan, J.W. J Comp Neurol, 1924. 38: p. 107-125; Kunitomo, K. 1918. 8: p. 161-204). It is the remnant of the nervous system that early in development provides innervation to the embryo's vestigial tail (or in the case of rodents, temporary innervation of caudal-most tail segments). At early stages, the presumptive FT is a fully differentiated spinal cord complete with dorsal root ganglia (Figure 1B, left). When the tail is reabsorbed, the cells of the filum undergo a process termed by Streeter "de-differentiation" (Figure 1B, right) resulting in a collagenous structure with a central canal lined by ependymal cells and ringed with a seemingly loosely organized collection of fibroblasts, neurons and glia. Paragangliomas and primitive neuroectodermal tumors have been shown arising from this structure which suggests the possibility that stem cells are present (Ashkenazi, E., et al. J Spinal Disord, 1998. 11(6): p. 540-2; Gagliardi, F.M., et al. Childs Nerv Syst, 1993. 9(1): p. 3-6; Kamalian, N., et al. J Neurol, 1987. 235(1): p. 56-9; Koeller, K.K., R.S. Rosenblum, and A.L. Morrison. Radiographics, 2000. 20(6): p. 1721-49). The FT is surgically easily accessible and is routinely sectioned in order to relieve tension on the spinal cord in cases in which it is tightly tethered to the spine and lacks sufficient freedom of movement. This condition is termed "tethered cord syndrome" (Bakker-Niezen, S.H., H.A. Walder, and J.L. Merx. Z Kinderchir, 1984. 39 Suppl 2: p. 100-3; Bode, H., et al. Klin Padiatr, 1985. 197(5): p. 409-14). Prior to the invention, the filum terminale has never been harvested as a potential source of neural stem cells.

FT is a safe and reliable source ofNSCs for the following reasons. It is surgically accessible and is an expendable nervous tissue. Human tissue is readily available from fetal tissue and from pediatric neurosurgery centers, which take biopsies of FT following surgical untethering of the spinal cord. Autologous transplants ofNSCs have the distinct advantage of avoiding immunologic rejection, which has been demonstrated to be a major problem with heterologous transplants into the nervous system (Barker, R.A. and H. Widner. NeuroRx, 2004. 1(4): p. 472-81; Linazasoro, G. Neurologia, 2003. 18(2): p. 74-100). NSCs have been identified in the mammalian CNS but current sources are difficult to access surgically and typically come from regions that are critical for normal function (e.g. spinal cord and lateral ventricle of the forebrain) (Alvarez-Buylla, A., D.G. Herrera, and H. Wichterle. Prog Brain Res, 2000. 127: p. 1-11; Alvarez-Buylla, A., B. Seri, and F. Doetsch. Brain Res Bull, 2002. 57(6): p. 751-8). Surgical disruption of these areas leads to profound neurological deficits. NSCs from the FT can be harvested throughout life and serve as a source for autologous NSCs, thereby avoiding the problem of immunologic rejection. Following isolation, culture, expansion, differention, autogolous FT-NCSs are transplanted into a subject for treatment of neurological disorders or injury.

Primary tissue is obtained from animal, e.g. rodent (rat, mouse), or human (e.g. fetal and postnatal human) FT. For example, fetal human tissue is obtained from discarded fetuses. Juvenile human FT is obtained during surgery for tethered cord resection. In addition, FT is obtained from postnatal rats. Postnatal rat FT has the same histological characteristics as human FT and has the advantage of being a readily-available, large-scale supply of tissue for further characterization of this class of NSCs.

The localization of FT-NSCs in tissue sections from postmortem human material of different ages from fetal to adult, provides information regarding the number of NSCs present at different stages and will lead to more precise dissections. FT is dissected, fixed and sectioned on a cryostat. NSCs are identified by staining tissue sections with antibodies directed against NSC markers such as nestin and Sox 2 (Bazan, E., et al. Histol Histopathol, 2004. 19(4): p. 1261-75).

### NSC Culture

NSCs of the invention are cultured for maintenance and for expansion prior to implantation into a subject.

FT are placed in tissue culture using various conditions including media and growth factors known in the art (e.g. DMEM/F12 with N2, EGF and bFGF + heparin) (Rajan, P. and E. Snyder. Methods Enzymol, 2006. 419: p. 23-52; Vescovi, A.L. and E.Y. Snyder. Brain Pathol, 1999. 9(3): p. 569-98). Neurospheres are isolated and passaged. They are identified using the non-limiting, immunological markers discussed above.

Procedures for separation include magnetic separation, using antibody-coated magnetic beads, affinity chromatography and panning using antibody attached to a solid matrix, e.g. plate, or other convenient technique. Other separation techniques include fluorescence activated cell sorters, which can have varying degrees of sophistication, such as multiple color channels, low angle and obtuse light scattering detecting channels, impedance channels, etc. Dead cells are eliminated using standard methods, e.g., by selection with dyes associated with dead cells (propidium iodide [PI], LDS). Any technique may be employed which is not unduly detrimental to the viability of the selected cells.

NSC lines are established and expanded for transplantation/recovery studies and in the case of human NSCs, to be developed as a source for therapeutic cell lines. Culture conditions for human NSCs are optimized to maximize growth rates and cell yields. By manipulation of media, growth factors, enzymes, etc. The growth rate of cell lines is measured by counting the number of neurospheres produced and by counting numbers of viable cells (Cardozo, D.L. Neuroscience, 1993. 56(2): p. 409-21).

### FT-NSCs differentiate into neurons and glia

Individual neurospheres are isolated and plated *in vitro* under various conditions that promote differentiation into neurons and glia (Reynolds, B.A., W. Tetzlaff, and S. Weiss. J Neurosci, 1992. 12(11): p. 4565-74; Reynolds, B.A. and S. Weiss. Science, 1992. 255(5052): p. 1707-10). To further characterize differentiation neurospheres are co-cultured with other cells, and pre-labeled with reagents such as the lipophilic membrane stain (such as DiI), carboxyfluorescein, or BrdU prior to exposure to differentiating conditions. This tracking strategy unambiguously establishes the neurospheres as the source of neurons and glia produced. Three basic approaches are used to characterize these cells: a. Plating on adhesive substrate in the presence of serum; b. Plating on an adhesive substrate following pretreatment with defined reagents known to promote a particular phenotype (e.g. the use of retinoic acid + sonic hedgehog protein to produce motor neurons); and c. co-culture of labeled neurospheres with cultured cells from target tissues such as muscle or cells from different regions of the CNS. The co-culture data establishes the influence of target tissue on FT-NSC fates and confirms that FT neurospheres form synaptic interactions with muscle cells and neurons.

Cell types derived from neurospheres are identified using antibodies directed against neurons and glia, including antibodies specific for neuronal and glial subtypes and antibodies that distinguish between immature and mature neuronal subtypes. These include: neural-specific enolase and -tubulin III (neurons); vimentin (neural precursors); GFAP (astrocytes); 01 (oligodendrocytes); choline acetyltransferase and MNR2 (motor neurons) (Gage, F.H., J. Ray, and L.J. Fisher. Annu Rev Neurosci, 1995. 18: p. 159-92; Schwartz, P.H., et al. J Neurosci Res, 2003. 74(6): p. 838-51; Schwartz, P.H., et al. Stem Cells, 2005. 23(9): p. 1286-94; Wichterle, H., et al. Cell, 2002. 110(3): p. 385-97).

Neurospheres exposed to reagents that promote the motor neuron phenotype are co-cultured with muscle cells (myotubes). The derived neurons are tested for their ability to establish synapses with muscle cells by staining the cultures with labeled -bungarotoxin which binds to the neuromuscular junction. Functional motor neurons are further evaluated using electron microscopy to identify the presence of the pre- and postsynaptic elements of functioning synapses (e.g. synaptic vesicles, postsynaptic density).

Morphologically identifiable neurons derived from neurospheres, are characterized using standard electrophysiological techniques to determine whether the cells have the physiological characteristics typical of neurons: measuring resting potential, the ability to produce action potentials, and responses to applied neurotransmitters. In the case of muscle co-culture, differentiated neurons are evaluated for muscle twitching response, both visual and electrophysiological methods (Cardozo, D.L. and B.P. Bean. J Neurophysiol, 1995. 74(3): p. 1137-48; Elkes, D.A., et al. Neuron, 1997. 19(1): p. 165-74).

### In vivo transplantation, integration and functional recovery experiments.

FT-NSC lines are evaluated for their ability to reintegrate into rodent host tissue and for their ability to produce functional recovery in human subjects as well as rodent models for spinal cord trauma and neurodegeneration. Art-recognized rodent models for lumbar spinal cord trauma are used for evaluation of FT-NSCs. These models include specific metrics for the degree of deficit and extent of functional recovery (Karimi-Abdolrezaee, S., et al. J Neurosci, 2006. 26(13): p. 3377-89; Kimura, H., et al. Neurol Res, 2005. 27(8): p. 812-9; Nakamura, M., et al. J Neurosci Res, 2005.81(4): p. 457-68; Vroemen, M., et al. Eur J Neurosci, 2003. 18(4): p. 743-51). GFP-labeled FT-NSCs are transplanted into the site of injury in rats that have undergone spinal cord trauma. Animals are tested for functional recovery then sacrificed and postmortem spinal cords are examined histologically for integration of transplanted cells.

Reintegration of FT-NSC cells is evaluated as follow, FT-NSC lines are established from a transgenic rat in which all CNS cells are labeled with green fluorescent protein (GFP) (Dombrowski, M.A., et al. Brain Res, 2006. 1125(1): p. 1-8). The GFP labeled FT-NSCs are concentrated and transplanted into spinal cord of normal rats. The ability of the transplanted cells to survive and integrate into host tissue is examined post-mortem using standard histological methods.

Human derived FT-NSC lines are evaluated by transplantation into human subjects as well as immunodeficient mouse lines (NOD-SCID). Transplanted cells are identified by staining for human-specific antigens such as SC101 and SC121 (Anderson, A.J., B.J. Cummings, and C.W. Cotman. Exp Neurol, 1994. 125(2): p. 286-95; Cummings, B.J., et al. Proc Natl Acad Sci U S A, 2005. 102(39): p. 14069-74).

Cells are also evaluated in rodent models for neurodegenerative disease (Kitamura, Y., et al. Jpn J Pharmacol, 2000. 84(3): p. 237-43; Orth, M. and S.J. Tabrizi. 2003. 18(7): p. 729-37; Springer, W. and P.J. Kahle. Curr Neurol Neurosci Rep, 2006. 6(5): p. 432-6). Animals are tested for functional recovery and postmortem for integration of transplanted cells into host tissue.

### Use of FT-NSCs to treat neurodegenerative diseases and neurotrauma.

Autologous FT NSCs are useful for the treatment of Parkinson's and other neurodegenerative diseases, as well as traumatic brain injuries. Studies suggest that dopamine producing cells can integrate into the striata of both murine Parkinson's models and human Parkinson's patients and alleviate symptoms of the disease. Prior to the invention, the lack of an abundant and readily accessible source of NSCs has meant that relatively few cells have been used in individual transplants. Moreover, prior transplants have used heterologous cells which, in spite of the "immunoprivileged" nature of the central nervous system, cause immune reactions that lead to rejection. The present invention solves both of these problems: every patient has an FT, therefore every patient has a ready source of autologous NSCs that are expandable and, under appropriate conditions, efficiently generate specific cell types, including dopamine neurons and cholinergic motomeurons, for autotransplantation. Because FT-NSCs eliminate fundamental constraints on autologous cell availability, the only limit to their therapeutic use is the variety of cell types to which they give rise. For example, FT-derived oligodendrocytes are used to treat demyelinating diseases such as multiple sclerosis, while FT-derived motoneurons are applicable to diseases of motoneuron loss such as amyotrophic lateral sclerosis or to spinal cord injuries.

### Harvest of Cells from human patients.

FT cells are harvested from patients using known methods. For example, surgery is used to cut and dissect the FT, as is done in cases of "tethered cord syndrome." Second, small amounts of tissue are harvested by needle aspiration. Given the high number of passages possible for these cells and the relatively small number required for a single injection (500,000 to 1,000,000), a single sample can yield enough cells for multiple injections. Using fluoroscopic or other guidance, multiple samples can be harvested during a single procedure.

Although the postnatal tissue was obtained from surgical specimens of TCS, all of the same experiments were also carried out using fetal derived HuFT NSCs and from post natal rat filum terminale. The results were consistent confirming the presence of NSCs in the filum terminale at all ages. In fact, immunostaining of normal 78 year old HuFT showed evidence of Nestin positive cells further supporting this assertion. The isolation and differentiation of NSCs from up to an 18 year old HuFT, suggests the possibility that these stem cells persist into adulthood.

FT is an untapped resource for autologous, expendable, accessible NSCs that profers the advantages of biosafety, histocompatibility and the lack of any deficits following its removal. NSCs from this tissue source are useful for treatment of nervous system trauma and degeneration.

The following reagents and methods were used to generate the data described in the examples below.

### In Vitro Differentiation

For non specific differentiation, single neurospheres were isolated using the help of a dissecting microscope for visualization, and plated on poly-L-lysine (0.01%, Sigma) and laminin (20 mg/ml, Sigma) coated glass coverslips in individual wells of 96 well culture dishes (Coming) in DMEM/F12 medium with 1% N2, 1 % penicillin-streptomycin, and 5-10% fetal bovine serum (Gibco). Medium was not changed for the rest of the experiment. Coverslips were processed 2-10 days later for imunocytochemistry. To confirm that the differentiated cells were derived from proliferative cells, neurospheres were incubated with tritiated thymidine (a gift from the Cepko lab, 5 mL per ml of media) for 8 hours. Subsequently, the neurospheres were visually isolated, washed X3 in stem cell media, and then differentiated as described above.
Directed differentiation into motor neurons first involved treatment of neurospheres with retinoic acid (RA, 2 mM, Sigma) and sonic hedgehog (Shh-N 500-100 nM from R&D systems, or Hh-Ag1.3, Curis) for 4-5 days. This treatment was performed in the stem cell media described earlier. Individual neurospheres were then isolated, and plated on poly-L-ornithine (0.01%, Sigma), collagen type I (0.01%, Sigma) and laminin (20 mg/ml, Sigma) coated glass coverslips in individual wells of 96 well culture dishes (Corning) in DMEM/F12 medium with 1% N2, 1% penicillin-streptomycin, 5% horse serum (Gibco), CNTF (25 ng/ml, Sigma), GDNF (25 ng/ml, Sigma), and BDNF (50 ng/ml) for 7-10 days. Three types of control experiments were performed. Neurospheres were treated with Shh-N alone without RA. Neurospheres were not treated with either RA or Shh-N but grown in the presence of BDNF, CNTF and GDNF. Lastly, some neurospheres were neither treated with Shh-N or RA, nor were they cultured in the presence of BDNF, CNTF or GDNF, but underwent non specific differentiation in 5-10% serum as described above. The controls too were cultured for 7-10 days in individual wells of 96 well culture dishes with coated coverslips as described earlier. All coverslips were then processed for immunocytochemistry.

To establish the presence of neuromuscular junction formation, individual neurospheres were treated with RA (2mM) and Shh-N (1000 nM) for 4-6 days and subsequently plated on muscle cultures in the differentiation media for MN growth and survival described above with CNTF, BDNF, and GDNF. Control cultures had untreated neurospheres plated onto the muscle cultures, or no neurospheres at all. After 21 days, cultures were incubated with fluorescent alpha bungarotoxin (2.5 mg/ml, labeled with alexa fluor 488) for 2.5 hours. They were then washed, fixed and processed for immunocytochemistry (the neuronal marker BTIII).

### Antibodies

Rabbit polyclonal antiserum to Nestin (1:400), goat polyclonal antibody to ChAT (1:100) and mouse monoclonal antibody to neuron specific enolase (1:1000) were obtained from Chemicon. Rabbit polyclonal Sox2 (1:1000) was from Sigma Abcam. Mouse monoclonal antibody to Vimentin was a gift from the Cepko laboratory. Mouse monoclonal CD133 (1:1000) was purchased from Miltenyi Biotec. Rabbit polyclonal antibody to GFAP (1:1000) was from Dako, and the mouse monoclonal to GFAP (1:1000) was from Sigma. Rabbit polyclonal to beta tubulin III was from Covance. Mouse monoclonal antibody to Tuj1 (1:1000) and Neu-N (1:1000) were also used as well as antibodies to Olig-2. The monoclonal antibodies to neurofilament, MNR2, Lim3 and Isl-1, and Pax6 were obtained from the Developmental Studies Hybridoma Bank developed under the auspices of the NICHD and maintained by The University of Iowa, Department of Biological Sciences, Iowa City, IA 52242. AF 488 conjugated donkey anti rabbit IgG, AF 488 conjugated donkey anti mouse IgG, AF 568 conjugated donkey anti goat IgG, AF 488 conjugated goat anti mouse IgG, and AF 568 conjugated goat anti rabbit IgG were the secondary antibodies obtained from the Alexa Fluor products from Invitrogen, all used at 1:1000.

### Immunocytochemistry

Immunocytochemistry was carried out with whole or differentiated neurospheres attached to glass coverslips. Coverslips were fixed in 4% formaldehyde (in PBS, pH 7.2) for 20-30 minutes, followed by 3 washes of 10 minutes each in PBS. The antibody dilutions were prepared in blocking solution (10% normal goat serum, 10% fish gelatin, 0.3% Triton X in 0.2% bovine serum albumin in PBS) and primary antibodies were incubated with the coverslips at their respective dilutions overnight (8 hours). This was followed by 3 washes in PBS prior to incubation with the appropriate secondary antibodies for 4 hours. After 3 further washes in PBS, Dapi (0.03 mg/ml) was incubated with the coverslips for 30 minutes. Coverslips were then washed 3 times (10 minutes each) one final time, and then mounted on glass slides with Vectashield as the mounting medium. The slides were visualized for immunofluorescence using a Zeiss photomicroscope. Approximate proportions of cells staining for a particular marker were determined by the average count of 4-5 20X fields. Dapi was used as the marker to count the total number of cells. If the number of cells was extremely large (>500), or they clustered together in some fields but were absent in others, the percentage of marker positive cells relative to Dapi was approximated.

Immunocytochemistry was used to establish the presence of various NSC, neural progenitor cell (NPC), neuronal and glial markers in HuFT derived undifferentiated neurospheres in vitro. All neurospheres (n = 13) stained positive for the NSC marker Nestin. In smaller neurospheres (< 100 microns), 100% of the cells expressed Nestin. However in larger neurospheres, the core appeared to be Nestin-negative. This core is likely a necrotic mass of cells. The neurospheres (n = 33) also contained cells positive for the neural progenitor markers Vimentin, CD 133 (n = 18), Olig2 (n =17) and Sox 2 (n = 17). The expression of NPC markers was variable between neurospheres. All the neurospheres tested (n=30) also differentially expressed the neuronal marker BTIII and the glial marker GFAP possibly heralding the varied patterns observed upon differentiation.

### Example 1: Production of Neurospheres from FT tissue.

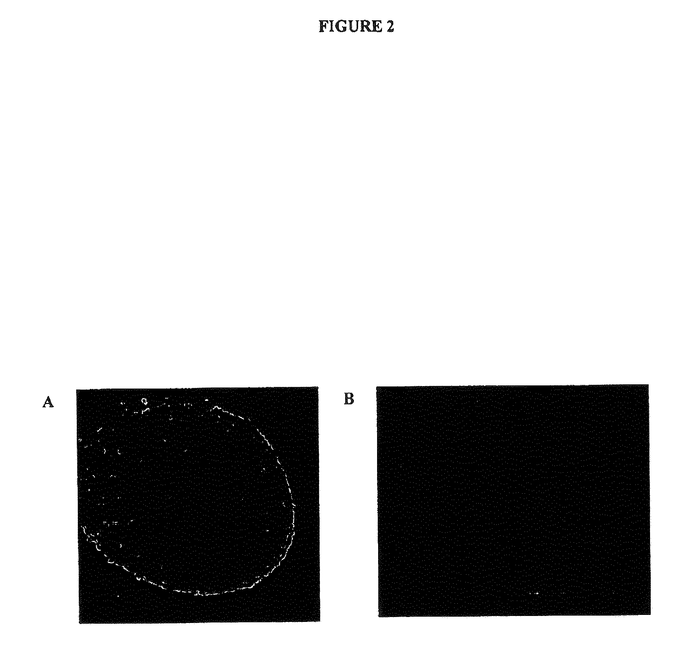

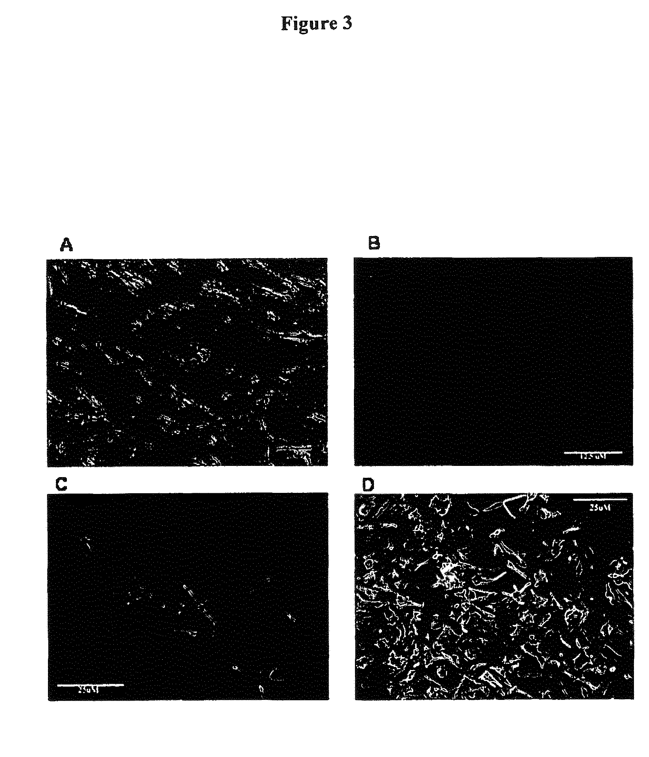

In 29 experiments, FT has been dissected and cultured from rats aged P4 to P19. Neurospheres were produced in 26/29 cultures (89.5%). Individual cultures have been passaged up to 18 times and 3 cell lines have been established and frozen. Human FT tissue has been isolated from 4 fetuses and from 12 postnatal surgeries aged 6 months to 18 years. Neurospheres were produced in 14/16 cultures (87.5%) and the cultures have been passage up to 6 times. The oldest tissue donor yielding neurospheres is 18 years old. Figs. 2A-D show the FT and caudal spinal cord and neurospheres derived therefrom. More than 20 human or rat neurospheres have been stained for nestin immunoreactivity (Figures 2, C and D). Every neurosphere tested has been nestin-positive. Figures 3A-D show differentiation of cells from FT neurospheres into neurons and glia. Individual neurospheres from rats and humans have been plated on various adhesive substrates including poly-1-lysine, laminin and collagen in the presence of serum. In all cases, FT-NSCs differentiated into neurons and glia (including astrocytes and oligodendrocytes) as determined by immunocytochemical criteria.

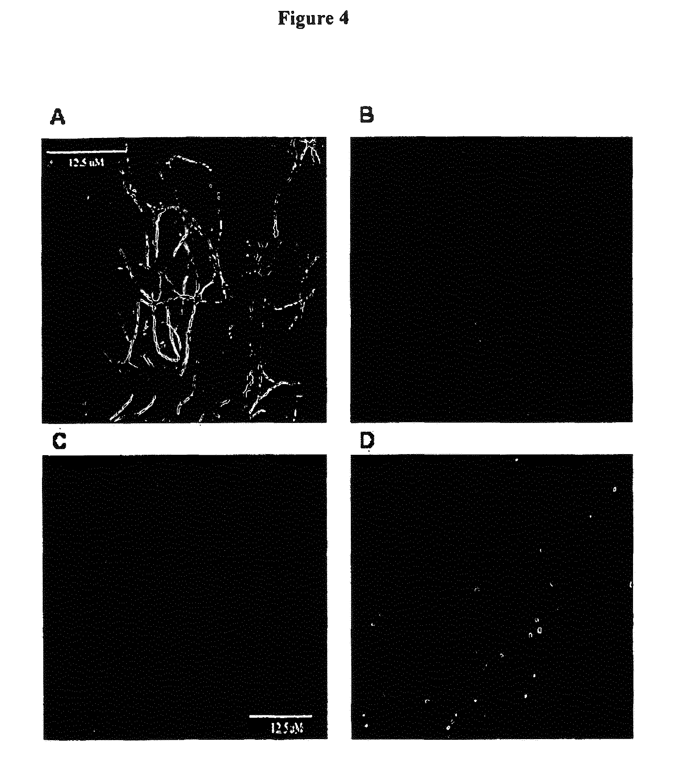

Single neurospheres have been incubated with retinoic acid and sonic hedgehog protein, and plated on an adhesive substrate in the presence of serum and neurotrophic factors. The FT-NSCs differentiated into morphologically identifiable neurons and stained for motor neuron markers including MNR2, LIM-3, ISL-1 and choline acetyltransferase (Figs. 4A-D). Neurospheres pre-labeled with DiI or with carboxyfluorescein differentiated into neurons when co-cultured with primary rat muscle cells. For establishment of motor neurons or whether they have formed neuromuscular junctions.

### Example 2: Culture of human FT and expansion and passaging of the derived NSCs.

Human fetal tissue, aged 14-21 weeks, was obtained after elective terminations of pregnancy. The spinal cord was rapidly dissected and placed in ice cold Hanks solution. Then, under microscopic visualization, the human FT was identified and dissected. Spinal nerve roots around the human FT were occasionally dissected and cultured separately as negative controls. Human post natal tissue, aged 6 months to 18 years, was obtained from children undergoing tethered cord release, a routine neurosurgical procedure for TCS. In these cases, the human FT was visually identified by the neurosurgeon with the assistance of a microscope, and its identity was confirmed with electrophysiological testing prior to removal. This tissue was transferred from the operating room to the laboratory in ice cold Hanks solution.

Once the fetal or post-natal FT tissue was obtained it was transferred into culture dishes (Corning), containing standard media, e.g., DMEM/F12 (1:1, Gibco), 1% N2 formulation (Gibco), 1% penicillin-streptomycin solution (Gibco), EGF (20 ng/ml, Gibco), bGFG (20 ng/ml, Gibco), LIF (10ng/ml) and collagenase type II 100U/ml with 3mM calcium (Gibco) and teased using a forceps and scalpel. The FGF was prepared in solution containing 8 mg/ml heparin (Sigma) for stability. The cultures were maintained in a humidified incubator at 37 degrees with 5% CO₂. After 24 hours, the tissue was partially digested by the collagenase and was triturated mechanically with a fire polished pipette for further dissociation. Primary stem cell proliferation was detected after 3-5 days *in vitro* and characterized by the formation of spheres of undifferentiated cells.

Tissue was obtained from 4 embryonic and 17 post natal sources. After 3-4 days *in vitro*, neurospheres were observed in 100% of the embryonic and 82% of the post natal cultures. Neurospheres are spherical, free floating, heterogenous aggregates ofNSCs that proliferate in culture whilst retaining the potential to differentiate into various neurons and glia. The number of neurospheres observed varied, ranging from 1 to more than 50 neurospheres per primary culture and did not correlate with the age of the donor. To demonstrate their capacity for proliferation and self renewal, they have been passaged up to 10 times and have been maintained them *in vitro* for up to 6 months. Eight lines have been frozen down, and tested for successful recovery of neurospheres.

The passaging frequency varied among primary cultures. Some cultures proliferated rapidly and required passaging every 10-14 days, others only required passaging every 3-4 weeks. Neurospheres were dissociated with 1X AccumaxTM (Innovative Cell Technologies) for 5-7 minutes and then triturated mechanically to achieve partial dissociation of neurospheres. After centrifugation (10 minutes, 1000 rpm), cells were resuspended in a 1:1 combination of fresh and conditioned medium. It was noted that if the neurospheres were dissociated into single cells during these passages, mortality was high, and occasionally 100%.

### Example 3: Spontaneous differentiation of human FT-NSCs into neurons and glia.

Some neurospheres adhered to the cultureware and appeared to spontaneously differentiate without the addition or removal of any factors from the medium. Single neurospheres from various donors successfully differentiated into neural progenitor cells (NPCs), neurons and glia in the presence of serum after the withdrawal of LIF, bFGF, and EGF. Individual neurospheres were plated in these differentiating conditions onto polylysine and laminin coated coverslips for 2-10 days. In all cases (n=50 experiments), neurospheres produced a varied assortment of NPCs, neurons and/or glia as identified by immunocytochemistry (Fig. 5 A & B). These included neuron specific enolase, neurofilament, neu-n, and beta tubulin III (neuronal markers), GFAP (astrocyte marker), 01 (mature oligodendrocyte marker), mushashi, vimentin, and sox-2 (NPC markers) (Fig. 5 A & B).

These differentiation experiments revealed heterogeneous neurosphere potentials both within and between the donor sources used (aged 6 months and 12 years) consistent with the observation of neurosphere heterogeneity in cellular composition and differentiation potential *in vitro.* The staining patterns observed also varied, with either cell clusters expressing a certain marker, or a more even interspersion of cells expressing different markers. When differentiated over 48 hours, a high proportion of cells (approximately 79%, n=8, SEM 0.08) double stained for both a neuronal and glial marker. However, after 7-10 days in differentiating conditions (n=7), the average proportion of double staining cells for neuronal and glial markers per neurosphere decreased to approximately 23% (SEM 0.09). Moreover, in these experiments, where after 7 days differentiated cells were stained for both a neuronal and glial marker, about half the neurospheres' potentials appeared to be either neuron dominant, or glia dominant with minimal or no double staining. In the other cases, varying proportions of both neurons and glia were generated, with some double staining. This variable potential persisted in neurospheres regardless of the source, and did not appear to be related to the age of the donor. Persistence of NPC marker staining was observed in approximately 98% of the differentiated cells even after 7-10 days (n=21, SEM 0.01). These cells frequently co-expressed neuronal or glial markers. This, combined with the double staining of neuronal and glial markers (that decreases with increasing differentiation time) indicates that the generated cells represent immature neurons and glia.

Human FT neurospheres proliferated and were passaged *in vitro.* These proliferative neurospheres differentiated into a collection ofNPCs, neurons and glia. To confirm that the differentiated neurons and glia were derived from proliferative cells, single neurospheres were treated with tritiated thymidine for 8 hours (n=4 experiments). The neurospheres were then removed from the tritiated thymidine containing environment, and differentiated as described earlier, for 7 days. In all 4 cases, 33-63% of the resulting neurons and glia had evidence of tritiated thymidine in their nuclei indicating that 8 hour exposure window had captured proliferative neurosphere cells in the S phase of the cell cycle. These cells had incorporated the radioactive nucleotide label whilst in the S phase and subsequently differentiated into neurons and glia (Fig. 5C).

Similarly, Rat FT neurospheres proliferate, can be passaged *in vitro* and that these proliferative neurospheres differentiate into a collection of NPCs, neurons and glia. To establish that the differentiated cells are derived from proliferative cells, tritiated thymidine was used to label the cells. In 5 experiments, neurospheres were treated with tritiated thymidine for 8 hours. They were then removed from the tritiated thymidine containing environment, washed, differentiated over 7 days in the standard conditions described above, and stained for BTIII and GFAP. In all 5 cases, 27-90% of the resulting neurons and glia had evidence of tritiated thymidine in their nuclei. These data indicate that the 8 hour exposure window captured some percentage of the NSCs in the 'S' phase of the cell cycle, and these proliferative cells incorporated the radioactive nucleotide label during this time. These cells subsequently differentiated into neurons and glia and were identified by the tritiated thymidine evident in their nuclei.

### Example 4: Directed differentiation of human FT-NSCs into motor neurons.

Prior to the invention, there were no previous reports of postnatal neurospheres generating motor neurons. Using a variation of Wichterle's previously described method of directed differentiation of embryonic stem cells into motor neurons (MNs), HuFT derived neurospheres as isolated and described herein were consistently induced to differentiate into MNs. First, single neurospheres were treated with retinoic acid and sonic hedgehog (Shh-N protein or a specific small molecule agonist of Shh signaling known as Hh-Ag1.3) for the induction of motor neuron progenitors (MNPs). Next, these individual neurospheres were plated on adhesive substrates in the presence of serum and three neurotrophic factors known to support MN growth and survival. The three neurotrophic factors were ciliary derived neurotrophic factor (CNTF), brain derived neurotrophic factor (BDNF), and glia derived neurotrophic factor (GDNF). After 7-10 days in these differentiating conditions the cells were analyzed by immunocytochemistry for the presence of MN specific markers. These included motor neuron restricted-2 (MNR2), Islet 1 (Isl1), Lim3 and choline acetyl transferase (ChAT). MNR2, first expressed during the final division of MNPs, is a committed determinant of MN identity. Isl1 and Lim3 are homeobox transcription factors associated with MN development. Isl1 is expressed by all classes of MNs. The differentiated neurospheres were tested for two progenitor markers that are known to be expressed in MNPs: the transcription factors Olig-2 and Pax6. In all experiments (n=16), different proportions of neurons expressed the MN or MNP markers described above (Fig. 5 D & E). This variability persisted even within the use of a single marker such as MNR2. The presence of homeobox 9 (HB9) a homeobox domain protein expressed selectively and consistently by somatic motor neurons, and Pax6 was confirmed by RT-PCR.

In past studies, embryonic stem cells (ESCs) readily differentiate into functional motor neurons when exposed to Hh-Ag1.3 and RA. The agonist is known to be more potent than the actual peptide, and has been used in preference to the peptide for the generation of MNs. Our data were consistent with this observation. In both experiments where HuFT neurospheres were differentiated after initial exposure to RA and Hh-Ag1.3, 100% of the neurons expressed MNR2. This observation, compared to the 5-40% of MNR2 positive cells generated after treatment with RA and Shh-N, indicated that Hh-Ag1.3 is a more potent agent for MN differentiation of HuFT neurospheres. Increasing the Shh-N concentration did not appear to alter the outcome. Shh-N alone also produced MNR2 positive cells with a similar range as those treated with Shh-N and RA. Untreated neurospheres cultured in the presence of GDNF, CDNF and BDNF, also consistently generated a variable proportion of MNR2 positive cells (Fig. 5 C). In multiple cases, cells that immunostained for MNR2 tended to cluster together. The use of RA and Shh-N for directed MN differentiation did not appear to be significantly superior to simply differentiating the neurospheres in the presence of CDNF, BDNF and GDNF (Fig. 5 D). Moreover, in 1 of 3 experiments, approximately 40% of cells from untreated neurospheres grown in serum only stained positive for MNR2. This observation, combined with the known heterogeneity of neurospheres, and the developmentally intended original function of the HuFT, indicated an innate potential of some HuFT NSCs to differentiate into MNs without requiring the ventralizing action of RA and inductive Shh-N signaling. The potent action of Hh-Ag1.3 appears to direct all HuFT neurosphere derived cells into MNs. HuFT derived NSCs treated with RA & Shh, and/or cultured in media containing CNTF,GDNF and BDNF, were capable of forming neuromuscular synapses with muscle fibers *in vitro* (n=16).

### Example 5: Isolation and characterization of self renewing Rat-FT derived neurospheres in response to EGF+FGF+LIF

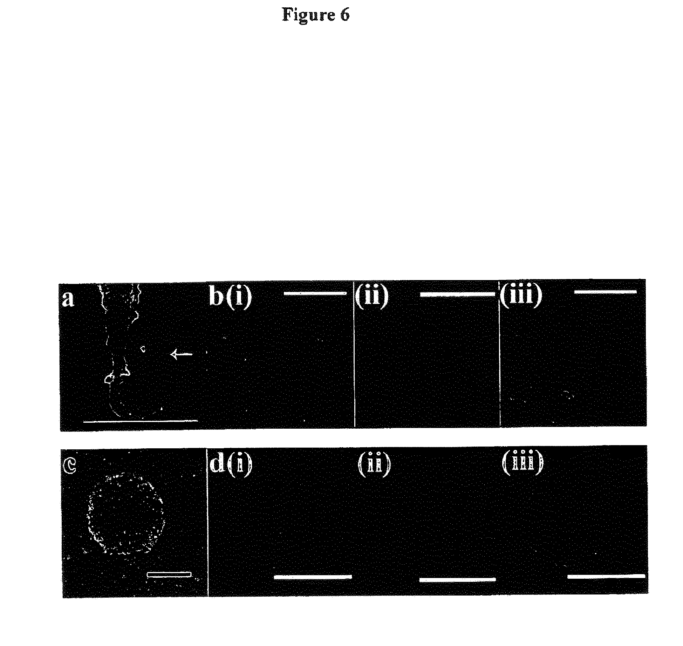

In order to determine whether there was any particular niche or specific location for the potential NSCs in the rat FT (rFT), the tissue was dissected and multiple sections of formaldehyde fixed tissue was stained for the NSC marker Nestin (Fig. 6A and B). The immunohistochemistry of these specimens revealed scattered, discrete Nestin positive cells with no apparent pattern of distribution.

The isolation of NSCs *in vitro* involved culturing collagenase dissociated primary tissue in standard stem cell medium (DMEM, F12, N2 supplement) containing bFGF (20 ng/ml), EGF (20 ng/ml) and human LIF (10 ng/ml). Previous studies have identified these mitogenic factors as successful stimulants to NSC proliferation, possibly with an additive effect. After 3-4 *days in vitro* neurospheres were observed in 31 out of the 34 primary cultures. These neurospheres were primarily free floating, and were identified by their spherical structure, phase bright appearance, regular cell membranes, and diffraction rings (Fig. 6 C). The number of neurospheres per primary culture varied from 10, to more than 40. This number did not appear to correlate with the age of the donor. To demonstrate their capacity for proliferation and self renewal, neurospheres were dissociated and passaged producing secondary spheres up to 19 times and have been maintained *in vitro* for up to 7 months.

Contrary to earlier thinking, neurospheres were not homogenous populations of NSCs, but have been shown to be a heterogeneous collection of different NSCs and neural progenitor cells (NPCs), likely with different potentials. Given this heterogeneous nature, the rFT derived neurospheres were characterized by using immunocytochemistry to determine the expression of various NSC, NPC, neuronal and glial markers (Table 1). Specifically, neurospheres were stained for the NSC marker Nestin (n=9), the NPC markers Sox2 (n= 8), Vimentin (n= 6), Olig- 2 (n= 3), and Musashi (n= 4), the neuron specific marker beta tubulin III (BT III, n=12), and the astrocytic marker glial fibrillary acidic protein (GFAP, n=12).

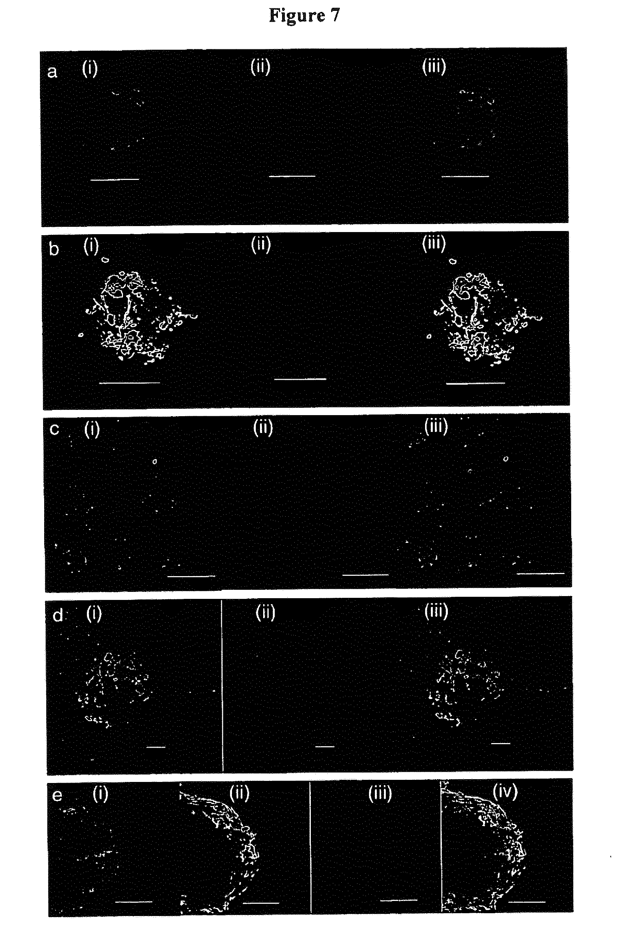

In all 9 cases, a varying proportion of cells were positive for Nestin. In 4/9 cases, 100% of the cells in the neurosphere expressed this NSC marker- this did not appear to be correlated to NS age or size (Fig. 2D). Staining for the neural progenitor markers was variable. In all 3 experiments, 100% of neurosphere cells stained positive for Olig-2 with some areas showing more intense staining (Fig.7 A). Regarding Sox-2 and Vimentin, although all neurospheres had some proportion of cells that stained positive for these markers (Fig. 7 B and C), this percentage varied from 40-100% for Sox-2, and 33-100% for Vimentin. Musashi staining was weak, with occasional hot-spots (Fig. 7 D). All neurospheres also expressed BTIII and GFAP- this expression was uniform in some neurospheres, but not in others (Fig 7 E). In the latter cases of differential expression, either BTIII or GFAP was expressed in the periphery, with the other marker expressed in the neurosphere core and vice versa.

### Example 6: Directed differentiation of RFT derived neurospheres to generate motor neurons (MNs)

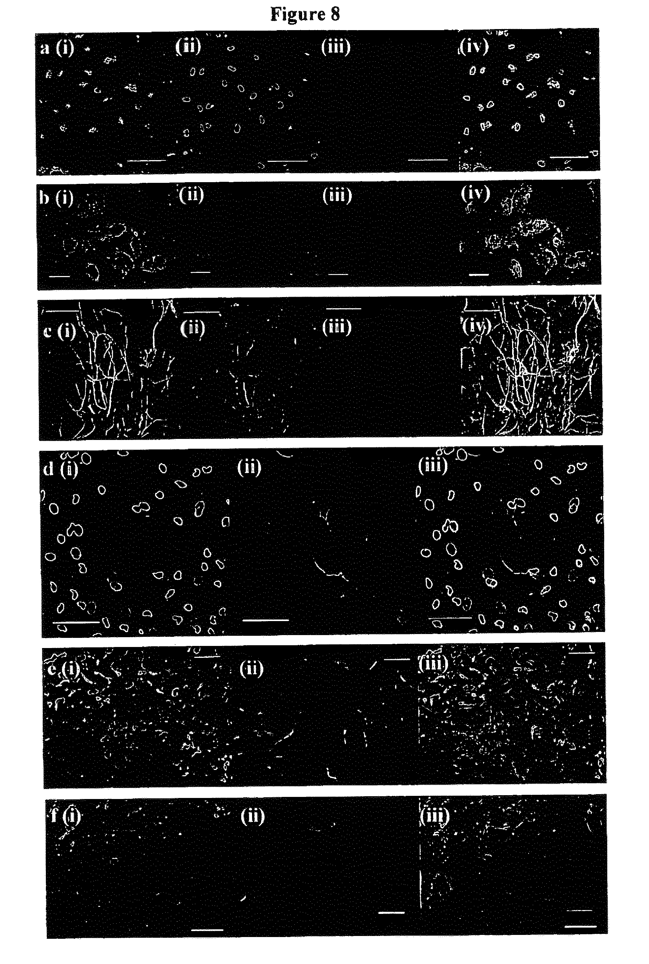

To consistently induce the generation of MNs from rFT derived neurospheres, single neurospheres were treated with retinoic acid (RA) and sonic hedgehog (Shh-N protein, or a specific small molecule agonist of Shh signaling called Hh-Ag1.3) for 4-5 days to induce MN progenitors. These treated individual neurospheres were then plated on adhesive substrates in the presence of serum and three neurotrophic factors known to support MN growth and survival. The neurotrophic factors used were ciliary derived neurotrophic factor (CNTF), brain derived neurotrophic factor (BDNF), and glia derived neurotrophic factor (GDNF). After differentiating the treated neurospheres in these conditions for 7-10 days, cells were analyzed by immunocytochemistry for the presence of MN specific markers (Figs. 8, 9). The markers used were motor neuron restricted-2 (MNR2), Islet 1 (Isl 1), Lim3 and choline acetyl transferase. MNR2, Isl1 and Lim3 were used by Wichterle et al., in their original paper describing the defined differentiation of embryonic bodies into MNS. Pax6 and Olig2 were used as markers to identify MN progenitor cells.

MNR2 is first expressed during the final division of motor neuron progenitors, and is a committed determinant of MN identity. Isl1 and Lim3 are two homeobox transcription factors associated with MN development. Isl1 is expressed by all classes of MNs. In all experiments (n=25), various proportions of differentiated neurons expressed the MN or MNP markers described above (Fig. 8). This variability persisted within the use of a single marker such as MNR2 (Fig. 9). In the 9 experiments where Hh-Ag1.3 was used, 95-100% of the differentiated neurons expressed MN markers such as MNR2, Isl1, Lim3 and ChAT. This agonist was more potent than the actual peptide, and has been used in preference to Shh-N for the generation of MNs. The data were consistent with the observation that neurospheres treated with Shh-N gave rise to differentiated neurons only 20-40% of which expressed MN markers. Increasing the Shh-N concentration did not appear to alter the outcome.

Three forms of control experiments were performed. The first involved treating neurospheres with Shh-N without RA (n=8) and then differentiating them in media containing serum and BDNF, CNTF and GDNF. The second, involved culturing untreated neurospheres in media containing serum and the three neurotropins (n=8). And the third involved differentiating untreated neurospheres in media containing serum without specific neurotrophic support (n=3). The former two conditions consistently generated a variable proportion of MNR2 positive cells (Fig. 8 E & F). In multiple cases, cells immunostaining for MNR2 tended to cluster together in islands, and were occasionally noted to be surrounded by glia. The use of RA and Shh-N for directed and consistent generation of MNs did not prove superior to simply differentiating the neurospheres in the presence of BDNF, CNTF and GDNF. However, when neurospheres were cultured in serum without neurotrophic support, the generation of MNs was inconsistent, where in only 1 out of 3 experiments approximately 40% of the cells expressed MNR2. These data, combined with the known heterogeneity of neurospheres, and the developmentally intended original function of the FT, indicate an innate potential of some rFT NSCs to differentiate into MNs without requiring the caudalizing action of RA or exogenous ventralizing Shh signaling. However, the use of Hh-Ag1.3 was found to be beneficial in increasing the MN yield in that most if not all of the neurosphere-derived cells were directed to generate MNs.

### Example 7: FT derived neurospheres are multipotent and differentiate into neurons and glia

Some neurospheres adhered to the cultureware and would spontaneously differentiate without the addition or removal of any factors from the medium. Studies were carried out to determine the conditions required to differentiate the rFT derived neurospheres into neurons and glia. After withdrawal of bFGF, EGF and LIF, in 34 experiments single neurospheres were plated onto various combinations poly-L-lysine and/or laminin coated coverslips and/or exposure to 5-10% serum. The neurospheres were subjected to these differentiating conditions for 7 days. Although the use of either adhesive substrate alone or serum alone was sufficient to initiate morphological differentiation, the addition of serum resulted in more rapid differentiation. In all cases, differented neurospheres expressed neuronal and glial markers including BTIII, neurofilament, O1, and GFAP (Table 1 and 2).

Once these conditions were established, the next 65 experiments further characterized the differentiating potential of the rFT neurospheres. In these experiments, the differentiating conditions involved withdrawal of all 3 growth factors, supplementation of the medium with 5-10% serum, and plating single neurospheres onto coverslips coated with poly-L-lysine and laminin. In 30 of these experiments, neurospheres were differentiated over 24 hours, and in 35 experiments the neurospheres were differentiated over 7-10 days. Consistent with the reported heterogeneity of neurospheres cultured from other regions of the mammalian CNS, rFT derived neurospheres had varied differentiation potentials.

Despite the variability, in all experiments, some proportion of NPCs, neurons and/or glia derived from each neurosphere were identified using immunocytochemical markers. These included Neuron specific enolase (NSE), NeuN, BTIII, GFAP, O1, Musashi, Vimentin and Sox2 (Table 1). Neurospheres differentiated over 24 hours (n= 9) had a high proportion of total cells that double stained for both neuronal and glial markers (approximately 70%, Table 3). This proportion decreased to an approximate average of 14.5 % after neurospheres were differentiated over 7-10 days (n=13, Table 3). Occasionally, after 7 days of differentiation, cells derived from neurospheres predominantly expressed either a neuronal or glial marker. In most cases however, no obvious predominance was observed. Despite the use of the same 2 markers in 14 experiments, differentiated neurospheres displayed a diverse array of BTIII and GFAP expression. This variation persisted in neurospheres both from the same source, and between different rFT sources.

The staining patterns between differentiated neurospheres were also variable. Sometimes, clusters of cells from a neurosphere would all stain positive for one particular marker and cells in other regions would express a different marker. More frequently however, cells staining for the different markers were interspersed together.

**Table 1**

| Antigenic Marker | Antigen Identified | Cell Type |
|---|---|---|
| Nestin | Intermediate filament | Stem cells |
| Musashi | RNA binding protein during development | Neural Progenitor Cells (gives rise to neurons and glia) |
| Sox 2 | Transcription factor | Neural Progenitor Cells (gives rise to neurons and glia) |
| Vimentin | Intermediate filament | Neural Progenitor (gives rise to neurons and glia) |
| Pax 6 | Homeobox domain (HD) gene transcription factor | Neuronal Progenitor Cells -expressed by undifferentiated in ventral region of neural tube & involved in Shh mediated control of neuronal identity; involved in spinal motor neuron identity |
| Olig-2 | Basic helix loop helix transcription factor (bHLH protein) | Motor neuron progenitor cells |
| GFAP | Intermediate filament | Mature astrocytes |
| O-1 | Cell surface marker (galactocerebroside) | Mature oligodendrocytes |
| Tuj1 / BT III | Intermediate filament | Neurons |
| Neuron Specific Enolase | Enolase enzyme | Neurons |
| | | |
| Neu-N | Neuronal nucleii | Neurons |
| | | |
| Neurofilament | Intermediate filament | Neurons |
| | | |
| MNR2/HB9 | Homeodomain protein (transcription factor) | Postmitotic motor neurons |
| | | |
| Isl 1 | LIM homeodomain protein (transcription factor) | Motor neurons |
| | | |
| Lhx 3/ Lim 3 | LIM homeodomain gene (transcription factor) | Motor neurons |
| | | |
| ChAT | Choline-acetyltransferase enzyme | Cholinergic neurons |

**TABLE 2**

| **Table 2: Various differentiation conditions attempted for rFT derived neurospheres** | | | | | |
|---|---|---|---|---|---|
| **Immunocytochemical marker** | | | | | |
| **Differentiating Conditions** | **O1** | **GFAP** | **BTIII** | **Neurofilament** | **Nestin** |
| Polylysine | + | + | + | + | 0 |
| Laminin | + | + | + | | 0 |
| Serum | + | + | + | + | 0 |
| Polylysine + laminin | + | + | + | + | 0 |
| Polylysine + serum | + | + | + | + | 0 |
| Laminin + serum | + | + | + | + | 0 |
| Polylysine + laminin + serum | + | + | + | + | 0 |

**TABLE 3**

| **Marker** | **Mean proportion of cells expressing the marker after 2 days of exposure to differentiating conditions (n, SEM)** | **Mean proportion of cells expressing the marker after 7-10 days of exposure to differentiating conditions (n, SEM)** |
|---|---|---|
| Beta Tubulin III | 0.83 (15, 0.05) | 0.48 (20, 0.07) |
| Neu-N | 0.63 (3, 0.27) | -- |
| Neuron specific enolase | 0.77 (3, 0.27) | -- |
| GFAP | 0.68 (9, 0.11) | 0.55 (13, 0.09) |
| O1 | 0.99 (5, 0.01) | 0.79 (4, 0.07) |
| Musashi | 0.92 (5, 0.05) | 1 (3, 0) |
| Sox2 | 0.77 (6, 0.02) | 1 (3,0) |
| Vimentin | 0.76 (3, 0.10) | 0.98 (5, 0.02) |
| Nestin | 0 (6,0) | --- |
| Proportion of total cells that double stained for a neuronal and glial marker | 0.70 (9, 0.12) | 0.15 (13, 0.09) |

### Example 8: FT-NSCs Generate Motor Neurons in vitro

Neural stem cells (NSCs) are undifferentiated cells in the central nervous system (CNS) that are capable of self-renewal and can be induced to differentiate into neurons and glia. Current sources of mammalian NSCs are confined to regions of the CNS that are critical to normal function and surgically difficult to access. This limits their therapeutic potential in human disease. It was unexpectedly discovered that the filum terminale (FT), a previously unexplored, expendable, and easily accessible tissue at the caudal end of the spinal cord, is a source of multipotent neurospheres in the mammal. In this study, a rat model was used to isolate and characterize the potential of these cells. Neurospheres from the rat FT (rFT) are amenable to *in vitro* expansion by a combination of epidermal growth factor (EGF), basic fibroblast growth factor (bFGF), and leukemia inhibitory factor (LIF). The proliferating cells formed neurospheres that were induced to differentiate into neural progenitor cells, neurons, astrocytes and glia by exposure to serum. Through directed differentiation using sonic hedgehog (Shh) and retinoic acid (RA) in combination with various neurotrophic factors, rFT derived neurospheres generated motor neurons (MN) *in vitro.*

The presence of multipotent NSCs has been demonstrated in multiple regions of the adult mammalian CNS in species ranging from rats to humans. These regions include the olfactory bulb, subependyma lining of the ventricles, hippocampus, cerebellum, spinal cord and retina. Current sources of mammalian NSCs are not ideal for transplantation therapy in human disease, because they are obtained from regions that are critical to normal function and that are difficult to access. Surgical disruption of these areas has lead to profound neurological deficits rendering it impractical to use them for harvesting autologous NSCs.

The FT is an excellent candidate as a source of autologous multipotent cells. It provides distinct advantages over the presently available sources in that it is an easily accessible and expendable tissue that persists in adults. Methods of the invention use the FT for autologous replacement therapy, thereby avoiding immunological problems.

Early in development, FT provides innervation to the presumptive tail of the embryo (or in rodents, temporary innervation of caudal-most tail segments). In adults, it is a vestigial remnant. Some humans are born with moveable tails, however, aberrant persistence of a tail likely represents failure of a developmental process. Embryogenesis of the human tail is first detected at the 3.5-5 mm stage (∼ 4 weeks). At the 11-15 mm stage (∼ 7 weeks), the coccygeal region shows more advanced development where the cord is differentiated into ependymal, mantal and marginal zones and has well-developed spinal roots entering it from respective dorsal root ganglia. There is no histologic indication at this time that this region will not go on to differentiate completely into the adult condition like the more cranial portions of the spinal cord.

As development continues, the coccygeal/tail portion of the spinal cord gets reabsorbed and the cells undergo a process termed by Streeter as "de-differentiation"(Streeter, G.L. 1919. Am J Anat 22:1-12). By the 30 mm stage (∼ 9.9 weeks), the coccygeal region of the spinal cord has changed significantly. The coccygeal spinal cord tissue reverts to an earlier embryonic type resulting in a collagenous structure with a narrow central canal lined by ependymal cells surrounded by a loosely organized collection of fibroblasts, neurons and glia (Streeter, G.L. 1919. Am J Anat 22:1-12). The marginal and mantle zones completely disappear, as do the last three coccygeal ganglia. The reabsorption occurs in a caudal to rostral direction and the resulting structure persists in adults as the FT: a slender prolongation of the caudal end of the spinal cord that anchors it to the coccyx.

Normally, the de-differentiated post natal FT is not interconnected with the rest of the nervous system, nor does it innervate the body. It is truly a vestigial remnant. In humans, it is routinely surgically transected in order to relieve tension on the spinal cord in cases where it is tightly tethered to the spine and lacks sufficient freedom of movement - a condition known as tethered cord syndrome. FT cells resemble an earlier embryonic cell type and retain the ability to re-differentiate into the multiple cell types present in the rest of the spinal cord.

In both humans and rats the FT is a collagenous structure that encloses the ventricular canal. Peri-ventricular ependymal cells and a loosely organized collection of fibroblasts, neurons and glia surround the canal. In rats, the FT neurons have been described as smaller than usual, and represent neurons in an early stage of commitment and differentiation. Paragangliomas and other primitive neuroectodermal tumors arise from the adult FT, again suggesting that NSCs are present. The FT is a source of multipotent cells.The use of a rat model permits the systematic, unlimited study of these cells in a controlled environment. In rodents, FT provides temporary innervation of the caudal most tail segments.

### Cell Culture

### Culture of rFT and derived neurospheres

*Primary Culture:* all procedures were conducted under sterile conditions. Postnatal rats (male and female, Sprague Dawley, Charles River) aged P2-P11 were anesthetized with isoflurane (Abbott) and sacrificed by cervical dislocation. The vertebral column was rapidly dissected in ice-cold Hanks solution. Under microscopic visualization, the rFT was identified and dissected. Each dissection was performed in less than 5 minutes to minimize cell death. Spinal nerve roots around the rFT were occasionally dissected and cultured separately as negative controls. The rFTs (usually 3 sibling rFTs per culture dish) were pooled and transferred into culture dishes (Coming), containing stem cell medium (SCM) (Weiss et al. 1996. J Neurosci 16:7599-7609; Carpenter et al. 1999. Exp Neurol 158: 265-278; Li et al. 2005a. Biochem Biophys Res Commun 326: 425-434; Kim et al. 2006. Exp Neurol 199: 222-235). This medium was made up of DMEM/F12 (1:1, Gibco), 1% N2 formulation (Gibco), 1% penicillin-streptomycin solution (Gibco), EGF (20 ng/ml, Gibco), bFGF (20 ng/ml, Gibco), and LIF (10ng/ml). The FGF was prepared in solution containing 8 mg/ml heparin (Sigma) for stability.

In order to dissociate the tissue, collagenase type II 100U/ml (Gibco) with 3mM calcium (Gibco) was added to SCM. Dissected tissue was then transferred this collagenase containing medium and teased using forceps and a scalpel. The cultures were maintained in a humidified incubator at 37 degrees with 5% CO₂. After 24 hours, the tissue was triturated mechanically with a fire polished pipette for further dissociation and left to remain in the collagenase containing SCM. Primary stem cell proliferation was detected after 3-5 days *in vitro* and characterized by the formation of spheres of undifferentiated cells (Reynolds, B.A. and Weiss, S. 1992. Science 255: 1707-1710).

*Passaging Cultures:* cultures were passaged every 2-3 weeks. Neurospheres were dissociated with 1X Accurnax^{™} (Innovative Cell Technologies) for 5-8 minutes and then triturated mechanically to achieve partial dissociation of neurospheres. In early experiments, when neurospheres were completely dissociated, few if any cells survived. After enzymatic dissociation, cells were centrifuged (10 minutes at 1000 rpm), and resuspended in a 1:1 combination of fresh and conditioned medium.

### Rat muscle culture.

P0-P7 rats were sacrificed and proximal limb muscles were rapidly dissected in ice-cold HBSS. The tissue was gently teased apart and then transferred to culture dishes containing media consisting of DMEM/F 12 (1:1), 1% N2 supplement and 1% penicillin-streptomycin. Collagenase type II (100 U/ml) with 3 mM calcium was added to this medium to disperse the muscle fibers into single cells. Dishes were placed in an incubator at 37° C with 5% CO₂ for 24 hours. After 24 hours, cultures were triturated with a fire polished Pasteur pipette to completely dissociate the tissue. Cultures were then centrifuged for 5 minutes at 1000 rpm. The pellets were washed X2 and then resuspended in medium containing DMEM/F 12 1:1. 1% N2 supplement, 1% penicillin-streptomycin, 10% fetal bovine serum. Cis-hydroxyproline (100 µg/ml) was added to the plating media to suppress fibroblast proliferation. Cells were plated at a density of ∼ 10⁶ cells/ml on coverslips coated with poly-L-lysine (0.01%) and laminin (20 µg/ml).

### Cell Differentiation

### In Vitro Differentiation

*Non specific differentiation with Serum:* single neurospheres were isolated using a dissecting microscope for visualization, and plated on poly-L-lysine (0.01 %) and laminin (20 µg/ml) coated glass coverslips in individual wells of 96 well culture dishes (Coming) in DMEM/F12 medium with 1% N2, 1% penicillin-streptomycin, and 5-10% fetal bovine serum (Gibco). Medium was not changed for the rest of the experiment. Coverslips were processed for imunocytochemistry after 24 hours, or 7-10 days later.

*Incubation with tritiated thymidine:* thymidine labeling experiments were conducted using the protocol of the Cepko laboratory (Dyer, M.A. and Cepko, C.L. 2000. Nat Neurosci 3:873-880). Neurospheres were incubated with ³H thymidine (NEN, 5 µCi/ml; 89 Ci/mmol) in SCM for 8 hours. The individual neurospheres were isolated, washed three times in SCM, and differentiated in serum as described above. After differentiation, coverslips were processed for immunocytochemistry. Prior to mounting the coverslips on slides, emulsifier oil was added to the coverslips and they were left in a dark room for 2 days. Emulsifier oil was removed and coverslips were washed with water. Developer was added for 4 minutes. Subsequently, the developer was aspirated and the coverslips were fixed in 4% paraformaldehyde for 20 minutes. Coverslips were then washed with water, mounted on slides with Vectashield and visualized under fluorescence (for immunocytochemistry) and brightfield microscopy (for tritiated thymidine incorporation).

*Directed Differentiation:* neurospheres were treated with retinoic acid (RA, 2 mM, Sigma) and sonic hedgehog (Shh) protein (Shh-N 400-1000 nM from R&D systems), or a small molecule agonist of sonic hedgehog signaling (Hh-Ag1.3, Curis), for 4-5 days using a modification of art-recognized methods (Wichterle et al. 2002. Cell 110:385-397; Soundararajan et al. 2006. J Neurosci 26: 3256-3268). This treatment was performed in SCM. Individual neurospheres were then isolated, and plated for 7-10 days on poly-L-ornithine (0.01%, Sigma), collagen type I (0.01%) and laminin (20 mg/ml) coated glass coverslips in individual wells of 96 well culture dishes (Coming) in DMEM/F12 medium with 1%N2, 1 % penicillin-streptomycin, 5% horse serum (Gibco), CNTF (25 ng/ml, Sigma), GDNF (25 ng/ml, Sigma), and BDNF (50 ng/ml). Four conditions were used: (1) neurospheres were treated as above; (2) as above but without RA; (3) as above without Shh or RA; (4) using serum alone, without Shh, RA or the 3 neurotropins. The coverslips were then processed for immunocytochemistry.

*Neuromuscular junction formation:* individual neurosphere were treated with RA (2mM) and Shh-N (600-1000 nM) for 4-6 days and subsequently plated on muscle cultures in the differentiation media for MN growth and survival described above. Two types of control cultures were used: 1) myocytes alone and 2) myocytes onto which untreated neurospheres were plated. After 6-21 days, cultures were incubated with fluorescent α-bungarotoxin (2 µg/ml), Molecular Probes alexa fluor 488) for 2.5 hours. They were then washed, fixed and processed for immunocytochemistry (the neuronal marker TUJ-1). Single neurospheres were labeled for 1 hour prior to co-culture with 1 µM Di-I or Di-D.

### Cell Markers

*Antibodies:* goat polyclonal antiserum against Nestin (1:50) from R&D systems. Goat polyclonal antibody against ChAT (1:100) and mouse monoclonal antibody against neuron specific enolase (1:1000) were obtained from Chemicon. Rabbit polyclonal against Sox2 (1:1000) was from Sigma and Abcam. Mouse monoclonal antibody against Vimentin was from Zymed. Rabbit polyclonal antibody against GFAP (1:1000) was from Dako, and mouse monoclonal against GFAP (1:1000) was from Sigma. Rabbit polyclonal against β-tubulin III (Tuj-1) was from Covance. The mouse monoclonal antibody against Tuj-1 (1:1000) and Neu-N (1:1000) were from Covance. Monoclonal mouse antibody against Olig-2, was prediluted, prior to use. Monoclonal antibodies against neurofilament; MNR2, Lim3 and Isl-1; and Pax6; were obtained from the Developmental Studies Hybridoma Bank developed under the auspices of the NICHD and maintained by the University of Iowa, Department of Biological Sciences, Iowa City, IA 52242. AF 488 conjugated donkey anti-rabbit IgG, AF 488 conjugated donkey anti-mouse IgG, AF 568 conjugated donkey anti-goat IgG, AF 488 conjugated goat anti-mouse IgG, and AF 568 conjugated goat anti-rabbit IgG were the secondary antibodies obtained from the Alexa Fluor products from Invitrogen, all used at 1:1000.

*Immunocytochemistry:* was carried out with whole or differentiated neurospheres attached to glass coverslips. Coverslips were fixed in 4% formaldehyde (in PBS, pH 7.4) for 20-30 minutes, followed by 3 washes of 10 minutes each in PBS. The antibody dilutions were prepared in blocking solution (10% normal goat serum, 10% fish gelatin, 0.3% Triton X in 0.2% bovine serum albumin in PBS) and primary antibodies were incubated with the coverslips overnight (8 hours). This was followed by 3 washes in PBS prior to incubation with the appropriate secondary antibodies for 4 hours. After 3 additional washes in PBS, coverslips were incubated in Dapi (0.03 mg/ml) for 30 minutes. Coverslips were then washed 3 times (10 minutes each), and then mounted on glass slides in Vectashield. The slides were visualized for immunofluorescence using a Zeiss photomicroscope or with confocal microscopy. Approximate proportions of cells staining for a particular marker were determined by the average count of 4-5 20X fields. Cell counts were based on nuclear staining using Dapi.

### Isolation and Characterization of rFT-Derived Neurospheres

*Isolation:* cells isolated from rFt were dissociated with collagenase and cultured in standard stem cell medium (DMEM, F12, N2 supplement) containing bFGF (20 ng/ml), EGF (20 ng/ml) and human LIF (10 ng/ml). After 3-4 *days in vitro* (DIV), neurospheres were observed in 31 out of the 34 primary cultures. These neurospheres were primarily free floating, and were identified by their spherical structure, phase bright appearance, and regular cell membranes. The neurospheres would initially appear as smaller clusters of 3-4 round cells that eventually grew into larger neurospheres. The size of these larger neurospheres ranged widely in size from about < 50 um to > 1 mm. Cell clusters of < 30 um were not counted as neurospheres. The number of neurospheres per primary culture varied from about 30, to more than 50. This number did not appear to correlate with the age of the donor rat. To demonstrate their capacity for proliferation and self renewal, neurospheres were dissociated and passaged up to 19 times. These cultures have been maintained *in vitro* for up to 7 months. Twelve cultures have been frozen, and two have been tested for viability and successfully recovered.

*Characterization:* neurospheres are not homogenous populations of NSCs, but are rather a heterogeneous collection of different NSCs and neural progenitor cells (NPCs), with varying differentiation potentials. The rFT-derived neurospheres were characterized using immunocytochemistry to determine the expression of various neural stem cell (NSC), neural progenitor cell (NPC), neuronal and glial markers (Table 1). Specifically, neurospheres were stained for the NSC marker Nestin (n=9); the NPC markers Sox2 (n= 8), Vimentin (n= 6), Olig-2 (n= 3), and Musashi (n= 4); the neuron specific marker β-tubulin III (Tuj-1, n=12); and the astrocytic marker glial fibrillary acidic protein (GFAP, n=12).

In all cases, a varying proportion of cells were positive for Nestin. In 4/9 cases, 100% of the cells in the neurosphere were Nestin⁺. This occurrence of Nestin staining did not appear to be correlated to neurosphere time in culture. Additionally, fixed whole mounts (n=1) and sectioned tissue (n=2) were stained for Nestin. Immunohistochemistry revealed Nestin⁺ cells. Staining for neural progenitor markers was variable. In 3/3 experiments, 100% of cells within the neurosphere stained positive for Olig-2 with some areas showing more intense staining. Although all neurospheres had some proportion of cells that stained positive for Sox-2 and Vimentin, this percentage varied from 40-100% for Sox-2, and 33-100% for Vimentin. Musashi staining was weak, with occasional clusters of high intensity staining.

Tuj-1⁺ and GFAP⁺ cells were present in all neurospheres (n=12). Every neurosphere contained some cells that were positive for both markers and this fraction varied greatly among neurospheres. There was spatial clustering of cells expressing the different markers. While this clustering was apparent in most neurospheres, the patterns were variable.

### Differentiation into Neurons and Glia.

Some neurospheres adhered to the cultureware and would spontaneously differentiate into cells having the morphological characteristics of neurons and glia without addition or removal of any factors from the medium. The conditions required to differentiate rFT derived neurospheres into neurons and glia were determined. After withdrawal of bFGF, EGF and LIF, single neurospheres were plated onto coverslips treated with 7 different combinations of adhesive substrates ± exposure to 5-10% fetal bovine serum as shown in Table 2. For each condition, 5 experiments were performed. After 7 days, cultures were stained for Tuj-1, neurofilament, O1, GFAP and Nestin. Although the use of either adhesive substrate alone or serum alone was sufficient to initiate morphological differentiation, the addition of serum resulted in more rapid differentiation. In all cases, cells derived from the neurospheres that expressed either neuronal or glial markers including Tuj-1, neurofilament, O1, and GFAP were detected.

All subsequent differentiation experiments (n=65) were conducted by withdrawing all 3 growth factors, supplementing the media with 5-10% fetal bovine serum, and plating single neurospheres onto coverslips coated with both poly-L-lysine and laminin. Cells were cultured in these conditions for 24 hours (n=30) or 7-10 days (n=35), and cultures were subsequently fixed for immunocytochemistry. Given the wide distribution of neurosphere sizes used in these experiments, the number of differentiated cells obtained ranged from about < 50 to > 5000 cells per neurosphere, which correlated with the size of the neurosphere initially plated. Larger neurospheres (usually > 100 um) were capable of generating > 5000 differentiated cells.

It was determined whether or not the neurospheres were capable of producing NPCs, neurons, astrocytes and oligodendrocytes. The immunocytochemical markers used to identify these cell types included Neuron specific enolase (NSE), NeuN, Tuj-1, GFAP, O1, Musashi, Vimentin and Sox2 (Tables 1 & 3). In each case, the cells derived from a single neurosphere were double-stained for two of these markers. Data from these experiments revealed that rFT-derived neurospheres had varied differentiation potentials. Neurospheres differentiated over 24 hours (n= 9) had a high proportion of cells that double stained for both neuronal and glial markers (Table 3). In the case of Tuj-1 and GFAP staining 69 ± 14% (n=5) of the cells were double stained for the two markers. After 7-10 days, the proportion of cells that double stained for both neuronal and glial markers decreased significantly (Table 3). In the case of Tuj-1 and GFAP staining, only 13 ± 10% (n=9) of the cells were double stained for the two markers. Variable expression of Tuj-1 and GFAP was observed in 14 experiments comparing differentiation after 24 hours to differentiation after 7-10 days. The varying proportions of Tuj-1⁺ and GFAP⁺ present in these rFT derived cell populations reflect the heterogenous differentiation potential of each neurosphere. This variation persisted in comparisons made between neurospheres obtained from both the same source, as well as from different rFT sources, regardless of the age of the rat.

On rare occasions, after 7 days of differentiation, > 85% of cells derived from a single neurosphere expressed *either* a neuronal or glial marker (n=2). In most cases, however, no obvious predominance was observed and varying proportions of *both* neuronal and glial cells were noted from the differentiation of a single neurosphere. NPC marker staining persisted even after exposure to differentiation conditions for 7-10 days (Table 3). In fact, the staining appears to slightly increase after 7-10 days as compared with staining at 24 hours for all the NPC markers used.

To establish that the differentiated cells are derived from proliferative cells, we labeled actively dividing cells with tritiated thymidine (3H). Neurospheres were treated with 3H for 8 hours (n=5). The neurospheres were then washed and differentiated over 7 days in the standard conditions described above. Derived cells were stained for Tuj-1 and GFAP. In all 5 cases, 27-90% of the cells identified immunologically as neurons and glia had incorporated tritiated thymidine into their nuclei (Fig. 12). This result demonstrates that the derived cells were the progeny of actively dividing cells.

### RFT-Derived Neurospheres Generate Motor Neurons (MNs)

In all experiments (n=25), various proportions of differentiated cells expressed MN or motor neuron progenitor markers described above. Fig. 12 shows the proportions of motor neurons, neurons and glia based on staining for MNR-2, Tuj-1 and GFAP.

Neurospheres treated with Shh-N gave rise to differentiated neurons, only 20-40% of which expressed MN markers MNR2, Isl1, Lim3 and ChAT (n=14). Increasing the Shh-N concentration from 400 to 1000 nM did not appear to alter the outcome. When Hh¬Ag1.3 (n=9, 1.5 µM) was used, 95-100% of the differentiated neurons expressed the MN markers. This result suggests that, at these concentrations, the Hh¬Ag1.3 agonist may be more effective than the actual Shh-N peptide, for the generation of MNs from FT derived neurospheres.

The differentiating conditions were varied to determine which factors were essential for generating MNs from FT: (1) Neurospheres were treated with Shh-N but without RA (n=8) and then differentiated them in media containing serum and BDNF, CNTF and GDNF; (2) Untreated neurospheres were cultured in media containing serum and the three neurotropins (n=8); (3) Untreated neurospheres were differentiated in media containing serum without the addition of neurotropic factors (n=3). As shown in Fig. 13, in conditions (1) and (2) neurospheres consistently generated a variable proportion of MNR2⁺ cells (5-67%).

In condition (3), the generation of MNs was inconsistent. In 1/3 cases, 40% of cells derived from the neurosphere expressed MNR2 and in 2/3 cases no cells were MNR2⁺. The use of RA and Shh-N for directed and consistent generation of MNs did not prove superior to simply differentiating the neurospheres in the presence of BDNF, CNTF and GDNF. However, Hh-Ag1.3 is beneficial in increasing the MN yield as described above and shown in Fig. 13. Given that FT is the vestigial remnant of the spinal cord, these results indicate a potential of some rFT NSCs to differentiate into MNs without the caudalizing action of RA or exogenous ventralizing Shh signaling.

The results of this study demonstrate that multipotent stem cells are present in the postnatal rFT. These cells exhibit two cardinal properties of NSCs: they are capable of self-renewal/expansion, and of differentiation into multiple cell types including neurons, astrocytes and oligodendrocytes. The ability of FT to generate MNs may be of particular therapeutic significance for neurodegenerative diseases such as ALS.

### The FT as an NSC Niche.

Methods of the invention have been used to determine that the FT histologic environment has many of the properties of a previously described CNS niche for NSCs such as the subventricular zone (SVZ) of the lateral ventricles. Cellular architecture in the SVZ consists of type A (neuroblasts), B (slowly proliferating GFAP⁺ neurogenic astrocytes), C (intermediate progenitor cells) and E (ependymal) cells. In this system, Type E cells line the ventricle and are occasionally displaced by B cells that weave between E cells to contact the ventricle. Type B cells lie towards the subventricular side of the E cells and ensheath A cells traveling to the olfactory bulb along a pathway known as the Rostral Migratory Stream. Type C cells are scattered along the chains of A cells.

These studies have shown that cell types in the FT include ependymal cells, neuroblasts, astrocyte-like cells, microglia, oligodendrocytes, neurons, fibroblasts, fat cells and ganglion cells. FT cells are loosely organized around ependymal cells that line the ventricular canal. FT ependymal cells often extend as rosettes beyond the ventricular lining forming extensive mosaics or rings of varying sizes. Without wishing to be bound by theory, the invention is based upon the surprising finding that FT ependymal cells are similar to the E cells of the SVZ. Moreover, like the B cells of the SVZ, some GFAP⁺ astrocyte-like cells in the FT have processes that interdigitate between the ependymal cells. Furthermore, the structure of these processes are similar to B cells ensheathing A cells in the SVZ. Data from studies performed using methods of the invention show that neurons found in the cranial portion of FT are similar to neuroblasts in morphology both in rats and humans. They often occur along tracts of nerve fibers and occasionally extend to the FT lateral margins. In certain embodiments of the invention, these cells are considered to be analogous to the A cells of the SVZ.

### Tumor Formation

CNS tumors are frequently found near neurogenic niches. Tumors within the spinal cord and FT constitute 4-10% of all CNS tumors. Paragangliomas and primitive neuroectodermal tumors such as ependymomas have an affinity for the filum terminale. Paragangliomas are neuro-endocrine tumors and ependymomas are tumors arising from the ependymal cells of the central canal. They account for 60% of all glial spinal cord tumors and are the most common intramedullary spinal neoplasm in adults; Myxopapillary ependymomas (tumors of ependymal glia in FT) constitute 13% of ependymomas, and have a distinct predilection for FT (Koeller, K.K. et al. 2000. Radiographics 20:1721-1749).

### Cell Identity IsDetermined Prior to The Differentiation Process

Neuronal and glial marker expression in single neurospheres that had been differentiated for 24 hours versus 7-10 days were compared. After 24 hours of the differentiation process, most cells expressed both neuronal and glial markers. In one aspect, this result reflects an unresolved cell fate early on in the differentiation process. After 7-10 days, most cells derived from a single neurosphere expressed either a neuronal or a glial marker with very few cells double staining for both. This result did not vary with donor age. Cells express a more committed cell fate with time, compared with a relatively ambiguous cell identity after 24 hours.

Cell identity was determined prior to the differentiation process. Neurospheres stained before differentiation, revealed different patterns of Tuj-1⁺ and GFAP⁺ cells despite identical treatment. Some cells within a neurosphere double stained for both markers, but most cells expressed only one marker, either Tuj-1 or GFAP. Cells positive for the same marker tended to cluster together spatially.

The neuronal or glial characteristics acquired prior to neurosphere differentiation predict the differentiation potential of each neurosphere. Temporary double staining during early stages of differentiation represent a point along the differentiation pathway where cell fate is ambiguous rather than undecided. Early staining patterns among neurosphere cells (GFAP⁺ or Tuj-1⁺) before differentiation implies that to manipulate a neurosphere toward a more neuronal or glial fate, requires culturing that neurosphere in different conditions from the outset.

### Neural Progenitor Cell (NPC) Markers Persist at 7-10 Days

NPC marker expression was high after 24 hours of differentiation, and even higher after 7-10 days. This was surprising, given that most cells cease double staining and express markers representing a more mature phenotype, *i.e*. a neuronal or a glial cell marker.

The persistence, and slight increase in NPC staining is attributed to two mechanisms. One explanation involves the change in metabolic activity of the cells with time. In one aspect, earlier in the differentiation process, some cells are highly metabolically active with a rapid protein turnover, preventing the detection of the NPC markers. After a few days of differentiation, as the turnover rate decreases, the protein levels build up, therefore enabling protein detection via immunocytochemistry. An alternate explanation is that after 7-10 days of differentiation, the cells are lineage specific NPCs, and therefore, express NPC markers in addition to neuronal or glial specific markers.

### RFT neurospheres have an innate potential to generate MNs

RFT-derived neurospheres generated MNs with and without exposure to RA and Shh, which have been used to differentiate embryonic stem cells into MNs *in vitro.* Rostral neural progenitors in embryonic bodies acquire a spinal positional identity in response to RA (a caudalizing signal), and subsequently attain a motor neuron progenitor identity in response to the ventralizing signals of Shh.

BDNF, CNTF and GDNF are neurotropins known to support MN growth and survival. RFT neurospheres treated with RA and Shh-N prior to differentiating them in the presence of serum, BDNF, CNTF, and GDNF generated 20-40% MNs. Neurospheres plated in serum with BDNF, CNTF and GDNF without RA or Shh-N treatment generated 5-67% MNs indicating that treating rFT neurospheres with Shh-N & RA is not more effective than plating them in the presence of BDNF, CNTF and GDNF for generating MNs. While RA and Shh are crucial in directing embryonic stem cell differentiation into MNs, these signals may not be as relevant to NSCs derived from the postnatal rFT, which may have already been, to some degree, caudalized and ventralized during embryonic development.

Some NSCs in FT may possess an ability to differentiate into MNs without requiring factors other than serum. When rFT derived neurospheres were plated in serum alone, 1/3 generated 40% MNs indicating that occasional MN expression can occur without specific intervention. In one aspect, this occurs because the FT is a vestigial remnant of the portion of the spinal cord that provided innervation to the embryonic tail (or, in the case of rodents, provide temporary innervation of the caudal most tail segments). When the developmental process of FT "de-differentiation" fails in humans, neonates can be born with moveable tails suggesting persistant MN innervation. In this situation, NSCs isolated from FT may possess an ability to generate cell types resident in the spinal cord such as MNs.

### Increasing MN Yield from rFT-Derived Neurospheres

In addition to Shh-N, Shh signaling is also activated by a small molecule agonist, Hh-Ag 1.3. For the generation of MNs from embryonic stem cells (ESCs), studies have used 300-500 nM Shh-N or 1-2 µM Hh-Ag 1.3 (Wichterle, H. et al. 2002. Cell 110:385-397; Harper, J.M. et al. 2004. Proc Natl Acad Sci U S A 101: 7123-7128; Miles, G.B. et al. 2004. J Neurosci 24:7848-7858; Li, X.J. et al. 2005. Nat Biotechnol 23: 215-221; Soundararajan, P. et al. 2006. J Neurosci 26:3256-3268). Although Wichterle et al., report identical results with Shh-N and Hh-Ag 1.3 at these concentrations, most studies have used 1 µM Hh-Ag 1.3 to generate MNs from ESCs. In rFT neurospheres, Hh-Ag1.3 was particularly effective in increasing the yield of generated MNs when compared to Shh-N. Nearly 100% of MNs were generated when Hh-Ag1.3 was used, while only 20-40% of MNs were generated when Shh-N was added. Hh-Ag 1.3 appears to be selectively efficient for increasing MN yield in rFT neurospheres versus ESC neurospheres. These results highlight one unexpected and superior property of rFT neurospheres.

### GFAP⁺ Cells Derived From FT Neurospheres

GFAP was used as an astrocytic marker, however, GFAP is also a marker for astrocyte-like adult stem cells. In adult mammals, neurogenic astrocytes have been identified *in vivo* in the SVZ of the lateral ventricle, and the subgranular zone of the dentate gyrus in the hippocampus. The characteristics and markers that distinguish neurogenic astrocytes from the vast population of non-neurogenic astrocytes remain unknown. GFAP⁺ cells differentiated from rFT are neurogenic and/or non-neurogenic astrocytes. In differentiation experiments, cells sometimes double stained for GFAP and a neuronal marker. Cells from neurospheres that have undergone directed MN differentiation, sometimes expressed both MNR2 and GFAP. The concurrent expression of a motor neuron marker with GFAP would be surprising if GFAP were solely a non-neurogenic astrocyte marker. Because GFAP is also a marker for astrocyte-like adult NSCs, double-stained cells could represent neurogenic astrocytes that are committed to an MN cell fate.

RFT neurospheres proliferate, can be passaged *in vitro* and differentiate into a collection of NPCs, neurons and glia. The discovery of multipotent cells within the mammalian CNS has had tremendous implications for therapeutic possibilities in many currently incurable CNS diseases including trauma, Alzheimer's, Parkinson's, Amyelotrophic Lateral Sclerosis (ALS), and multiple sclerosis. The discovery of FT as a source of multipotent cells opens up new possibilities in the field of autologous transplantation therapy for these neurological diseases.

### Example 9: The Postnatal Human filum terminale is a Source of Autologous Multipotent Neurospheres Capable of Generating Motor Neurons

Methods of the invention were used to isolate human NSCs from donors up to 18 years of age. These cells gave rise to neurospheres which proliferated over extended periods of time in culture. The neurospheres have been induced to differentiate into neurons and glia. Additionally, they have been induced to form motor neurons capable of innervating striated muscle *in vitro.* This is the first human source of multipotent CNS cells that is both accessible and expendable, and the first report of motor neurons from human neurospheres derived from postnatal tissue. The invention provides for an autologous cell-based transplantation therapy that circumvents immunological rejection.

A source of autologous NSCs was sought that was expendable in humans. The *FT* was chosen as a point of focus because of its unique developmental history and its propensity to produce paragangliomas and neuroectodermal tumors. The *FT* is a slender prolongation of the caudal end of the spinal cord (approximately 15 cm in the adult) that anchors the cord to the coccyx at the base of the spine (Fig. 1A). It is the remnant of the nervous system that early in development provides innervation to the embryo's vestigial tail or in the case of tailed vertebrates, temporary innervation of the caudal-most tail segments. At early stages, the presumptive *FT* is a differentiated spinal cord complete with three additional dorsal root ganglia (C3-C5) (Fig. 1A). When the tail is reabsorbed, the cells of *FT* undergo a reversion to an earlier embryonic state by a process termed by Streeter as "de-differentiation" (Fig. 1A, right). The result is an elongated structure having a central canal which narrows to the point of disappearing caudally, lined by ependymal cells and ringed with a seemingly loosely organized collection of fibroblasts, fat cells, rosettes of non-ciliated ependymal cells, neuroblasts, neurons and glia. This local environment has many of the properties of other CNS regions that produce NSCs. The *FT* is surgically accessible and is routinely surgically cut in order to relieve traction on the spinal cord in cases of 'tethered cord syndrome' (TCS) in which the cord lacks sufficient freedom of movement.

*FT* tissue was obtained from human fetuses and from postnatal surgeries. *FT* was dissected from electively terminated fetuses aged 14 to 21 weeks (Fig. 1B). Postnatal tissue, aged 6 months to 18 years, was obtained from neurosurgical cases of TCS. There was no ambiguity concerning the tissue source, as all surgical *FT* specimens were obtained from within the dural sheath and the *FT's* identity was confirmed using clinical electrophysiology prior to resection. Sections were made from 3 postnatal *FTs* and tested for the presence of the NSC marker Nestin. Immunohistochemistry of 3 *FT* specimens aged 8 months to 5 years revealed the presence Nestin⁺ ependymal cells as well as dispersed neural progenitor cells (Fig. 1C).

Neurospheres, which are free floating aggregates of proliferating cells, were isolated from *FT*. Tissue was obtained from 4 fetal and 17 postnatal donors. Primary tissue was enzymatically dissociated and cultured using standard conditions to promote neurosphere growth. After 3-4 days *in vitro* (DIV), we observed neurospheres in 100% of fetal and 82% of postnatal cultures (Fig. 10F). The neurospheres started as small clusters of cells and grew into spheres from about 25µm to > 500 µm in diameter. The number of neurospheres isolated varied from about 1 to more than 50 neurospheres per primary culture and this abundance was independent of donor age. To demonstrate their capacity for proliferation and self-renewal, neurospheres have been successfully dissociated and passaged up to 10 times and have been maintained *in vitro* for 6 months, the longest period attempted. Eight cultures have been frozen, and one has been tested for viability and successfully recovered.

In order to characterize the neurospheres and their potential to produce neurons and glia, neurospheres were tested for expression of various immunocytochemical markers. Immunocytochemistry was performed on single neurospheres and for each assay, neurospheres came from more than one donor. Neurospheres were tested for various neural stem cell (NSC), neural progenitor cell (NPC), neuronal and glial markers. All neurospheres were Nestin⁺ (n = 13) (Fig 10A). In smaller neurospheres (< 100 µm), 100% of cells were Nestin⁺, while in larger neurospheres the core appeared to be Nestin⁻. All the neurospheres tested also contained cells positive for the NPC markers Vimentin (n = 33), CD 133 (n = 18), Olig-2 (n =17) and Sox-2 (n = 17) (Fig. 10B-D). The expression pattern and proportion of NPC⁺ cells was variable among neurospheres. We stained 42 neurospheres for Tuj-1, which recognizes the neuronal protein β-tubulin III, and for the astrocyte marker GFAP (Fig. 10E). Tuj¬1⁺ and GFAP⁺ cells were present in all neurospheres. Additionally, every neurosphere contained some cells that were positive for both markers and this double-positive fraction varied greatly. As shown in Figure 10E, there was spatial clustering of cells expressing the different markers. While clustering was apparent for most neurospheres, the patterns were variable.

In order to test the ability of neurospheres to produce differentiated cell types, single neurospheres were plated onto poly-L-lysine and laminin coated coverslips using the media described above in which the growth factors were replaced by 5% fetal bovine serum. The neurospheres were derived from two donors, aged 6 months and 12 years. The cultures derived from individual neurospheres, were examined using immunocytochemistry 2-10 days after plating. To confirm that the differentiated cells were derived from proliferating cells, single neurospheres were incubated with tritiated thymidine for 8 hours (n=4). The neurospheres were subsequently differentiated and examined after 7 days. In all cases, a significant proportion (33- 63%) of the resulting neurons and glia had incorporated the radioactive nucleotide into their nuclei (Fig. 5C).

Cultures were stained with antibodies against the neuronal markers Neuron Specific Enolase (n=4), and Tuj-1 (n=19); the astrocyte marker GFAP (n=11); the oligodendrocyte marker O1 (n=5); and the NPC markers Vimentin (n=9), CD 133 (n=4), Olig-2 (n=3) and Sox-2 (n=8). The number of differentiated cells obtained from each culture ranged from about < 50 to > 5000 cells which correlated with the size of the plated neurosphere (n=50). There was great variability in the proportions of cell types produced by each neurosphere which did not correlate with neurosphere size or donor age. Two days after plating, 79 +/- 8% (n=8) of the cells derived from a neurosphere that double-stained for a neuronal marker and either GFAP or O1 (Fig 5B). After 7-10 days, double staining decreased to 23 +/- 9% (n=7) and in approximately half of the cultures, either neurons or astrocytes predominated, suggesting variable potentials among neurospheres. There were no Nestin⁺ cells at 10 days following differentiation (n=3), however 98% of cells remained positive for a NPC marker (n=21) in addition to a neuronal or a glial marker (Fig 5A), indicating that the cells were not yet fully differentiated.

Since *FT* represents a vestigial portion of the spinal cord, we determined whether *FT* neurospheres were capable of generating spinal cord motor neurons (MNs) that could be used in cell replacement strategies in cases of spinal cord trauma or MN degeneration. To produce MN progenitors, single neurospheres were treated for 4-6 days with 2 µM retinoic acid and 0.4-1 µM sonic hedgehog protein (Shh-N) (Wichterle, H. et al. 2002. Cell 110:385-97). Neurospheres were subsequently plated on adhesive substrate in the presence of 5% horse serum and 3 neurotrophic factors known to promote MN growth and survival: ciliary-derived neurotrophic factor, brain-derived neurotrophic factor, and glia-derived neurotrophic factor (Zurn, A.D. et al. 1996. J Neurosci Res 44:133-41). After 7-10 days, the fraction of MNs produced by each neurosphere was determined using immunocytochemistry for the MN marker Motor Neuron Restricted-2 (MNR¬2) (Jessel, T.M. Nat Rev Genet 1:20-9). Neurospheres treated with Shh-N produced 20 +/- 12% MNR-2⁺ cells (n=6) and increasing the Shh-N concentration did not appear to affect the proportion of MNs (Fig. 5D). Interestingly, when a small molecule Shh-N agonist, Hh-Ag1.3 (1.5 µM) (Frank-Kamenetsky, M et al. 2002. J Biol 1:10;Harper, J.M. et al. 2004. PNAS USA 101:7123-8) was included in the treatment, 100% of cells expressed MNR-2 (n = 2) suggesting that Hh-Ag1.3 may be more effective at inducing MN differentiation. When untreated neurospheres were plated with the 3 neurotrophic factors (n=4) we detected 5-50% MNR2⁺ cells and 2/3 untreated neurospheres plated in serum alone yielded positive cells (1% and 40%) suggesting that some neurospheres can produce MNs in the absence of added factors. To confirm the MN identity, we tested for additional MN markers: Lim-3 (n=3), Islet-1 (n=1), and choline acetyltransferase (n=2) (Fig. 5E)(Oda, Y. and Nakanishi, I. 2000. Histol Histopathol 15, 825-34; Arber, S. et al. 1999. Neuron 23, 659-74; Pfaff, S. L. et al.1996. Cell 84, 309-20; Tsuchida, T. et al. 1994. Cell 79, 957-70). In all cases, the results were similar to those for MNR-2 with respect to the proportion of stained cells. Additionally 4/4 neurospheres expressed Homeobox-9, a homeobox domain protein expressed selectively by somatic motor neurons as determined by RT/PCR (Pfaff, S.L. et al. 1996. Cell 84: 309-20).

To determine whether the cells characterized as MNs by immunocytochemical criteria were capable of innervating muscle, neurospheres were added to striated muscle cultures from postnatal rat. Neurospheres were treated with retinoic acid and Shh-N as described above and subsequently a single neurosphere was added to each muscle culture. To confirm that the neurons in the co-culture were derived from plated neurospheres, 4 neurospheres were pre-incubated with a lipophilic carbocyanine dye, DiD, for 2.5 hours prior to plating. In all cases we detected DiD⁺ cells that had the morphological characteristics of neurons. There were no neurons detectable by phase microscopy or Tuj-1 staining in muscle cultures without added neurospheres (n=12). After co-culture with neurospheres for 6-21 days, cultures were incubated with fluorescent α-Bungarotoxin to detect clustering of nicotinic acetylcholine receptors at neuromuscular junctions. All of the co-cultures showed evidence of neuromuscular junctions by this criterion (n=18). Figure 11B shows a culture stained for both α-Bungarotoxin and for Tuj-1 to demonstrate a neuromuscular junction and the neuron providing the innervation. Control cultures containing only muscle fibers did not contain neuromuscular junctions (n=12).

Although most FTs were obtained from surgical specimens of TCS, virtually indistinguishable results were obtained with *FTs* derived from terminated fetuses and from extensive experiments with FT from postnatal rats. These data indicate that the presence of multipotent cells in *FT* reflects the normal condition. The *FT* is a source of autologous, expendable, accessible multipotent cells for use in cases of nervous system trauma or degeneration. The isolation and differentiation of these cells from donors up to 18 years of age, indicates that they persist into adulthood.

### Isolation and differentiation of neurospheres from the HuFT

Human fetal tissue (aged 14-21 weeks) was dissected in ice cold Hanks buffer (Fig 1b) as was human pediatric tissue. The tissue was dispersed in DMEM/F12 (1:1, Gibco) with collagenase type II 100U/ml (Gibco) and maintained in standard stem cell medium of DMEM/F 12, 1 % N2 formulation (Gibco), 1% penicillin-streptomycin solution (Gibco), EGF (20 ng/ml, Gibco), bGFG (20 ng/ml, Gibco), LIF (10ng/ml)(Weiss, S. et al. 1996. J Neurosci 16, 7599-609). Every 2-4 weeks, neurospheres were passaged following dissociation with Accumax (Innovative Cell Technologies). Differentiation of neurospheres was induced by withdrawal of growth factors, addition of 5-10% serum to the medium and plating on coverslips coated with poly-1-lysine and/or laminin. For differentiation into MNs, neurospheres were treated with RA (2 uM, Sigma) and 0.4-1 µM Shh-N (R&D systems) or 1.5 µM Hh-Ag 1.3 (Curis) for 4-6 days followed by plating for 7-10 days on coverslips coated with poly-L-ornithine, laminin and collagen in Neurobasal media containing BDNF, CNTF and GDNF (Sigma) (Wichterle, H. et al. 2002. Cell 110, 385-97).

### OTHER EMBODIMENTS

While the invention has been described in conjunction with the detailed description thereof, the foregoing description is intended to illustrate and not limit the scope of the invention, which is defined by the scope of the appended claims. Other aspects, advantages, and modifications are within the scope of the following claims.

### Embodiments of the invention will now be described in the following numbered paragraphs:

1. A composition comprising a population of filum terminate (FT) neural cells enriched for neural stem cells (NSCs).
2. The composition of paragraph 1, wherein said population comprises a neurosphere or a neurosphere initiating stem cell.
3. A composition comprising a population of isolated FT cells comprising at least 10% neural stem cells.
4. A composition comprising a population of isolated FT cells comprising at least 30% neural stem cells.
5. A composition comprising a population of isolated FT cells comprising at least 90% neural stem cells.
6. A method of isolating FT-NSCs from a post-natal animal, comprising providing a FT tissue from said animal, dissociating said FT tissue to obtain neurospheres, and recovering nestin-positive NSCs.
7. A composition comprising an isolated NSC obtained by the method of paragraph 6.
8. A method of augmenting or restoring neurological function in a subject comprising administering to said subject a population of isolated FT cells.
9. A method of treating a neurological disorder, comprising harvesting FT tissue from a subject, culturing FT cells ex vivo to produce an enriched population of isolated FT-NSCs, and administering to said subject said an enriched population of isolated FT-NSCs.
10. The method of paragraph 9, wherein said neurological disorder comprises an injury or a degenerative condition.
11. The method of paragraph 9, wherein said neurological disorder comprises an injury or diminuition of function of the brain or spinal cord.
12. The method of paragraph 9, wherein said subject is diagnosed as having suffered a stroke or suspected of having suffered a stroke.
13. A cell line comprising multipotent descendant cells from an FT-NSC.
14. A method of expanding FT-NSCs from a post-natal animal, comprising providing isolated FT-NSCs from said animal and culturing said FT-NSC under conditions that allow for proliferation or differentiation of said FT-NSC
15. The method of paragraph 6, wherein said FT tissue is obtained by needle aspiration.

It should be understood that various changes and modifications to the presently preferred embodiments described herein will be apparent to those skilled in the art. Such changes and modifications can be made without departing from the spirit and scope of the present invention and without diminishing its intended advantages. It is therefore intended that such changes and modifications be covered by the following claims.

## Claims

1. An *in vitro* or *ex vivo* method comprising
(a) culturing an isolated FT cell population under conditions that promote
(i) proliferation of FT cells; and/or
(ii) differentiation of FT cells.

2. The method of claim 1, wherein the method comprises exposing the FT cell population to a signal promoting proliferation and/or to a signal promoting differentiation,
(a) optionally wherein exposing the FT cell population to a signal promoting proliferation comprises culturing the FT cell population in the presence of a mitogenic factor, optionally wherein the mitogenic factor is EGF, FGF, or LIF,
(b) optionally wherein exposing the FT cell population to a signal promoting differentiation comprises
(i) culturing the FT cell population on an adhesive substrate in the presence of serum;
(ii) culturing the FT cell population on an adhesive substrate in the absence of a mitogenic factor; and/or
(iii) culturing the FT cell population in the presence of a reagent promoting differentiation, optionally wherein the reagent promoting differentiation is retinoic acid (RA), a Sonic Hedgehog (Shh) signaling agonist, or a neurotrophic factor.

3. The method of claim 1 or 2, wherein the FT cell population comprises a neural stem cell.

4. The method of any one of claims 1-3, wherein the differentiation of FT cells results in the generation of a neural progenitor cell, a neuronal cell, and/or a glial cell, optionally wherein
(i) the neuronal cell is a motorneuron, a dopaminergic neuron, a cholinergic neuron, or a GABAergic neuron;
(ii) the neuronal cell expresses Olig-2, Tuj1, neuron-specific enolase, Neu-N, Neurofilament, HB9, Isl1, Lim3, and/or ChAT;
(iii) the glial cell is an astrocyte, an oligodendrocyte, or an ependymal cell; and/or
(iv) the glial cell expresses GFAP and/or O-1.

5. An isolated FT cell population for use in the treatment of a central nervous system (CNS) disorder or in the treatment of a peripheral nerve disorder in a subject.

6. The FT cell population of claim 5, wherein the FT cell population comprises disaggregated or dissociated FT cells.

7. The FT cell population of claim 5 or claim 6, wherein the FT cell population comprises neural stem cells, neural progenitor cells, and/or differentiated cells, optionally wherein the differentiated cells comprise differentiated neural or glial cells, optionally wherein the differentiated cells comprise ependymal cells, neuroblasts, astrocyte-like cells, microglia, oligodendrocytes, neurons, fibroblasts, fat cells, and/or ganglion cells,
optionally wherein the ependymal cells comprise non-ciliated ependymal cells.

8. The FT cell population of any one of claims 5-7, wherein the FT cell population is formulated for administration to a subject,
(a) optionally wherein the FT cell population is autologous to the subject,
(b) optionally wherein the FT cell population is allogeneic to the subject.

9. The FT cell population of any one of claims 5-8, wherein the CNS disorder is a neurodegenerative disease, an acute brain injury, or a CNS dysfunction, and, optionally, wherein the CNS disorder is Alzheimer's Disease, Multiple Sclerosis, Huntington's Disease, Amyotrophic Lateral Sclerosis, Parkinson's Disease, stroke, head injury, cerebral palsy, depression, epilepsy, or schizophrenia.

10. An isolated FT cell population for use in an *ex vivo* or *in vitro* method for generating
(i) an FT-derived neural stem cell population comprising at least 10% neural stem cells;
(ii) an FT-derived neurosphere; and/or
(iii) an FT-derived differentiated cell.

11. The isolated FT cell population of claim 10, wherein the method comprises exposing the isolated FT cell population ex *vivo* or *in vitro* to a signal promoting proliferation and/or to a signal promoting differentiation,
(a) optionally wherein exposing the isolated FT cell population to a signal promoting proliferation comprises culturing the isolated FT cell population in the presence of a mitogenic factor,
optionally wherein the mitogenic factor is EGF, FGF, or LIF;
(b) optionally wherein exposing the isolated FT cell population to a signal promoting differentiation comprises
(i) culturing the isolated FT cell population on an adhesive substrate in the presence of serum;
(ii) culturing the isolated FT cell population on an adhesive substrate in the absence of a mitogenic factor; and/or
(iii) culturing the isolated FT cell population in the presence of a reagent promoting differentiation, optionally wherein the reagent promoting differentiation is RA, a Shh signaling agonist, or a neurotrophic factor.

12. The isolated population of FT cells of claim 11, wherein the FT-derived neural stem cell population, the FT-derived neurosphere, or the FT-derived differentiated cell is for use in the treatment of a CNS disorder in a subject,
(a) optionally wherein the CNS disorder is a neurodegenerative disease, an acute brain injury, or a CNS dysfunction, and, optionally, wherein the CNS disorder is Alzheimer's Disease, Multiple Sclerosis, Huntington's Disease, Amyotrophic Lateral Sclerosis, Parkinson's Disease, stroke, head injury, cerebral palsy, depression, epilepsy, or schizophrenia,
(b) optionally wherein the population of FT cells is autologous to the subject,
(c) optionally wherein the population of FT cells is allogeneic to the subject.

13. A pharmaceutical composition comprising an isolated FT cell population.

14. The pharmaceutical composition of claim 13 wherein the isolated FT cell population comprises neural stem cells, neural progenitor cells, neural cells, glial cells, ependymal cells, non-ciliated ependymal cells, neuroblasts, astrocyte-like cells, microglia, oligodendrocytes, neurons, fibroblasts, fat cells, and/or ganglion cells, or any combination thereof,
(a) optionally wherein the FT cell population comprises *in vitro* or ex *vivo* cultured FT cells,
(b) optionally wherein the FT cell population comprises FT cells that have been exposed *in vitro* or *ex vivo* to a signal promoting proliferation and/or to a signal promoting differentiation.

15. The pharmaceutical composition of any one of claims 13 or 14, wherein the pharmaceutical composition is for use in the treatment of a CNS disorder in a subject,
(a) optionally wherein the CNS disorder is a neurodegenerative disease, an acute brain injury, or a CNS dysfunction, and, optionally, wherein the CNS disorder is Alzheimer's Disease, Multiple Sclerosis, Huntington's Disease, Amyotrophic Lateral Sclerosis, Parkinson's Disease, stroke, head injury, cerebral palsy, depression, epilepsy, or schizophrenia,
(b) optionally wherein the population of FT cells is autologous to the subject,
(c) optionally wherein the population of FT cells is allogeneic to the subject.
